# EUROPEAN PATENT APPLICATION

(11) **EP 2 311 811 A1**
(43) Date of publication of application: **20.04.2011**
(21) Application number: 09797722.7
(22) Date of filing: 17.07.2009
(51) Int. Cl.: C07D 221/20, A61K 31/435, A61K 31/438, A61P 3/04, A61P 3/06, A61P 3/08, A61P 3/10, A61P 9/12, C07D 471/10

(54) **NOVEL SPIRO COMPOUND, AND PHARMACEUTICAL PREPARATION COMPRISING THE SAME**

(30) Priority: 18.07.2008 US 81788
(71) Applicant: Kowa Company, Ltd., Nagoya-shi, Aichi-ken 460-8625 (JP)
(72) Inventor: NAKASHIMA, Hisashi, Higashimurayama-shi Tokyo 189-0022 (JP); OGAMINO, Takahisa, Higashimurayama-shi Tokyo 189-0022 (JP); ARAKI, Takaaki, Higashimurayama-shi Tokyo 189-0022 (JP); GOMI, Noriaki, Higashimurayama-shi Tokyo 189-0022 (JP); KANEKO, Yasushi, Higashimurayama-shi Tokyo 189-0022 (JP); ABE, Kazutoyo, Higashimurayama-shi Tokyo 189-0022 (JP); OHGIYA, Tadaaki, Higashimurayama-shi Tokyo 189-0022 (JP); SHIBUYA, Kimiyuki, Higashimurayama-shi Tokyo 189-0022 (JP)
(74) Representative: HOFFMANN EITLE
(86) International application number: PCT/JP2009/003370
(87) International publication number: WO 2010/007794

(57) **Abstract**

It is to provide a novel compound useful for preventing and/or treating diabetes, insulin resistance, diabetes complication, obesity, dyslipidemia, hypertension, fatty liver, or metabolic syndrome.
A spiro compound represented by the following general formula (1) or salt thereof, or their solvate.

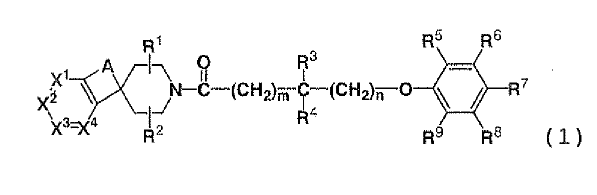

## Description

### Technical Field

The present invention relates to a novel spiro compound having an 11β-hydroxysteroid dehydrogenase 1-inhibitory activity and a medicine comprising the same.

### Background Art

11β-hydroxysteroid dehydrogenase (hereinafter, abbreviated as 11β-HSD)1 is an enzyme that converts in cells an inactive form of glucocorticoid (cortisone or 11-dehydrocorticosterone) into an active form of glucocorticoid (cortisol or 11β-corticosterone), and is found to be expressed on the liver, central nerves and the like as well as subcutaneous fat and visceral fat (non-patent documents 1 and 2). Meanwhile, in cells, enzyme 11β-HSD2 is also present that converts an active form of glucocorticoid into an inactivated form. An active form of glucocorticoid is converted in cells from inactive precursor by the action of 11β-HSD1, thereby exercises its effect. Glucocorticoid has been reported to involve in adipocyte differentiation and to inhibit glycolipid metabolism that is helped by insulin (non-patent document 3). 11β-HSD1 activity and expression: level in adipose tissues positively correlate with body-mass index (BMI) or insulin resistance (non-patent document 4). Further, it is reported that a transgenic mouse over-expressing 11β-HSD1 specifically in adipose tissues exhibits a phenotype comprising a combination of major factors of metabolic syndrome, such as visceral fat accumulation, insulin resistance, dyslipidemia, hypertension and fatty liver (non-patent documents 5 and 6). By contrast, it is reported that, in an 11β-HSD1 knockout mouse, an inactive form cannot be converted to an active form and as a result, the induction of the group of gluconeogenic enzymes attributable to the burden of high-fat food does not occur in the liver, which acts suppressively on hyperglycaemia due to obesity (non-patent document 7). It is also reported that decreased blood triglyceride, elevated HDL cholesterol, and improved insulin resistance were observed (non-patent document 8). From these findings, active form of glucocorticoid produced excessively by 11β-HSD1 is considered to cause the onset of a metabolic disease such as diabetes, insulin resistance, diabetes complication, obesity, dyslipidemia (hyperlipidemia), hypertension, and fatty liver, or a metabolic syndrome pathology which comprises a series of these metabolic diseases. Therefore, a selective inhibitor of 11B-HSD1 is believed to be useful for treating or preventing the above pathologies.

Heretofore, many compounds have been reported for the purpose of inhibiting 11β-HSD1 activity. The examples of reported compounds include compounds having a spiro structure (patent documents 1 to 5), adamantane derivative (patent document 6), sulfonamide derivative (patent document 7), pyrazole derivative (patent document 8), isoxazole derivative (patent document 9), triazole derivative (patent document 10), tetrazole derivative (patent document 11), pyridine derivative (patent document 12), pyrimidine derivative (patent document 13), piperidine derivative (patent document 14), pyridazine derivative (patent document 15), pyrrolidine derivative (patent document 16), thiazole derivative (patent document 17), thiophene derivative (patent document 18), lactam derivative (patent document 19) and the like.

On the other hand, spiroindane and spiroindene compounds having a similar structure with the compound of the present invention have been reported (patent document 20), while it is related to a selective antagonist of oxytocin effective to preterm birth or dysmenorrhea. Inhibition of 11β-HSD1 activity is not described nor suggested.

### [Prior Art Documents]

### [Patent Documents]

Patent Document 1: WO2005/110992
Patent Document 2: WO2006/040329
Patent Document 3: WO2006/053024
Patent Document 4: WO2006/055752
Patent Document 5: WO2005/046682
Patent Document 6: WO2005/108368
Patent Document 7: WO2006/134467
Patent Document 8: WO2006/132436
Patent Document 9: WO2006/132197
Patent Document 10: WO2007/007688
Patent Document 11: WO2007/029021
Patent Document 12: WO2006/010546
Patent Document 13: WO2006/000371
Patent Document 14: WO2005/046685
Patent Document 15: WO2007/003521
Patent Document 16: WO2004/037251
Patent Document 17: WO2006/051662
Patent Document 18: WO2004/112779
Patent Document 19: WO2006/049952
Patent Document 20: WO1994/07496

### [Non-patent Documents]

Non-patent Document 1: J.Mol. Endocrinol., 37:327-340 (2006)
Non-patent Document 2: Endcr. Rev., 25:831-866 (2004)
Non-patent Document 3: Rinsho-i, viol.30, No.9, 1782-1787 (2004)
Non-patent Document 4: J.Clin. Endocrinol. Metab., 88:2738-2744 (2003)
Non-patent Document 5: Science 294: 2166-2170 (2001)
Non-patent Document 6: J.Clin. Invest. 112:83-90 (2003)
Non-patent Document 7: Proc. Natl. Acad. Sci. USA 94:14924-14929 (1997)
Non-patent Document 8: J. Biol. Chem., 44 41293-41301 (2001)

### Summary of the Invention

The object of the present invention is to provide a novel compound that inhibits 11β-HSD1 selectively, and is useful as a medicine.

### Means to solve the problem

The present inventors made a keen study to find a compound that selectively inhibits 11β-HSD1. Consequently, the present inventors have found that a compound having a spiro skeleton represented by the following formula (1) is a compound that inhibits 11β-HSD1 selectively and thus completed the present invention. More specifically, the present invention relates to [1] a spiro compound represented by the following general formula (1) or salt thereof, or their solvate:

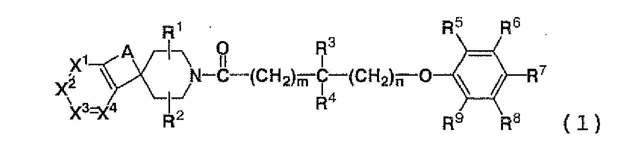

(wherein all of X¹, X², X³ and X⁴ are C-R¹⁰, or one of X¹, X², X³ and X⁴ is N, and the rest is C-R¹⁰, A represents the following formula (2) or (3):

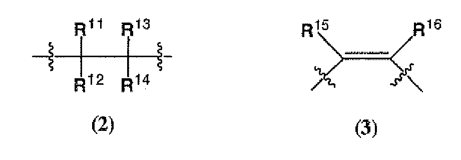

R₁, R₂, R₃, and R₄ are same or different and are a hydrogen atom, C₁₋₆ alkyl group, halo C₁₋₆ alkyl group, C₂₋₆ alkenyl group, C₂₋₆ alkynyl group, C₃₋₈ cycloalkyl group, C₁₋₆ alkoxy group, C₁₋₆ alkoxy-C₁₋₆ alkyl group, C₆₋₁₀ aryl group, C₆₋₁₀ aryloxy group, or 5 - to 10-membered heteroaryl group; R⁵, R⁶, R⁷, R⁸, R⁹ and R¹⁰ are same or different, and are a hydrogen atom, halogen atom, C₁₋₆ alkyl group, halo C₁₋₆ alkyl group, C₂₋₆ alkenyl group, C₂₋₆ alkynyl group, C₃₋₈ cycloalkyl group, C₁₋₆ alkoxy group, halo C₁₋₆ alkoxy group, C₁₋₆ alkanoyl group, formyl group, nitro group, amino group, mono C₁₋₆ alkylamino group, diC₁₋₆ alkylamino group, cyano group, hydroxy group, carboxyl group, C₁₋₆ alkoxycarbonyl group, carbamoyl group, C₆₋₁₀ aryl group, 5- to 10-membered heteroaryl group, C₁₋₆ alkylthio group, C₁₋₆ alkylsulphonyl group, C₆₋₁₀ arylthio group, or C₆₋₁₀ arylsulphonyl group; or two adjacent R⁵∼R⁹ may be bond to form C₁₋₆ alkylenedioxy group; R¹¹, R¹², R¹³, R¹⁴, R¹⁵ and R¹⁶ are same or different, and are a hydrogen atom, halogen atom, C₁₋₆ alkyl group, halo C₁₋₆ alkyl group, C₂₋₆ alkenyl group, C₂₋₆ alkynyl group, C₃₋₈ cycloalkyl group, C₆₋₁₀ aryl group, hydroxy group, or C₁₋₆ alkoxy group, or R¹¹ and R¹², or R¹³ and R¹⁴ may together bond with a same oxygen atom; m and n represent an integer of 0 to 6, and the sum of m and n represents an integer of 0 to 6)

[2] The spiro compound or salt thereof, or their solvate according to [1], wherein at least one of R⁵, R⁶, R⁷, R⁸ and R⁹ is a halo C₁₋₆ alkyl group or C₆₋₁₀ aryl group;

[3] The spiro compound or salt thereof, or their solvate according to [1], wherein the compound represented by the general formula (1) is a compound selected from the group consisting of:
1-[spiro(indene-1,4'-piperidine)-1'-yl]-2-[2-(trifluorometh yl)phenoxy]ethanone,
1-[spiro(indene-1,4'-piperidine)-1'-yl]-2-(o-tolyloxy)ethan one,
2-(2-ethylphenoxy)-1-[spiro(indene-1,4'-piperidine)-1'-yl]e thanone,
2-(2-iodophenoxy)-1-[spiro(indene-1,4'-piperidine)-l'-yl]et hanone,
ethyl
2-[2-oxo-2-[spiro(indene-1,4'-piperidine)-1'-yl]ethoxy]benz oate,
1-[spiro(indene-1,4'-piperidine)-1'-yl]-2-[3-(trifluorometh yl)phenoxy]ethanone,
2-(4-methoxyphenoxy)-1-[spiro(indene-1,4'-piperidine)-1'-yl ]ethanone,
2-(biphenyl-4-yloxy)-1-[spiro(indene-1,4'-piperidine)-1'-yl ]ethanone,
2-(2,3-dichlorophenoxy)-1-[spiro(indene-1,4'-piperidine)-1' -yl]ethanone,
1-[2,3-dihydrospiro(indene-1,4'-piperidine)-1'-yl]-2-[2-(tr ifluoromethyl)phenoxy]ethanone,
1-[2-hydroxy-2,3-dihydrospiro(indene-1,4'-piperidine)-1'-yl ]-2-[2-(trifluoromethyl)phenoxy]ethanone,
1'-[2-[2-(trifluoromethyl)phenoxy]acetyl]spiro(indene-1,4'-piperidine)-2(3H)-one,
1'-[2-[2-(trifluoromethyl)phenoxy]acetyl]spiro(indene-1,4'-piperidine)-3(2H)-one,
1-[spiro(cyclopenta[b]pyridine-7,4'-piperidine)-1'-yl]-2-[2 -(trifluoromethyl)phenoxy]ethanone,
1-[5,6-dihydrospiro(cyclopenta[b]pyridine-7,4'-piperidine)-1'-yl]-2-[2-(trifluoromethyl)phenoxy]ethanone,
1-[2-methylspiro(indene-1,4'-piperidine)-1'-yl]-2-[2-(trifl uoromethyl)phenoxy]ethanone,
1-[2-ethylspiro(indene-1,4'-piperidine)-1'-yl]-2-[2-(triflu oromethyl)phenoxy]ethanone,
1-[2-propylspiro(indene-1,4'-piperidine)-1'-yl]-2-[2-(trifl uoromethyl)phenoxy]ethanone,
1-[2-isopropylspiro(indene-1,4'-piperidine)-1'-yl]-2-[2-(tr ifluoromethyl)phenoxy]ethanone,
1-[2-cyclopropylspiro(indene-1,4'-piperidine)-1'-yl]-2-[2-( trifluoromethyl)phenoxy]ethanone,
1-[2-phenylspiro(indene-1,4'-piperidine)-1'-yl]-2-[2-(trifl uoromethyl)phenoxy]ethanone,
1-[2-chlorospiro(indene-1,4'-piperidine)-1'-yl]-2-[2-(trifl uoromethyl)phenoxy]ethanone,
1-[2-bromospiro(indene-1,4'-piperidine)-1'-yl]-2-[2-(triflu oromethyl)phenoxy]ethanone,
1-[3-methylspiro(indene-1,4'-piperidine)-1'-yl]-2-[2-(trifl uoromethyl)phenoxy]ethanone,
1-[2-methyl-2,3-dihydrospiro(indene-1,4'-piperidine)-1'-yl] -2-[2-(trifluoromethyl)phenoxy]ethanone, and 1-[2-ethylspiro(indene-1,4'-piperidine)-1'-yl]-2-methyl-2-[ 2-(trifluoromethyl)phenoxy]propane-1-one.

[4] a pharmaceutical composition consisting of a spiro compound or salt thereof, or their solvate according to any one of [1] to [3], and a pharmaceutically acceptable carrier;

[5] an inhibitor of 11β-hydroxysteroid dehydrogenase 1, comprising the spiro compound or salt thereof, or their solvate according to any one of [1] to [3] as an active ingredient;

[6] an agent for preventing and/or treating diabetes, insulin resistance, diabetes complication, obesity, dyslipidemia, hypertension, fatty liver, or metabolic syndrome, which agent comprises the spiro compound or salt thereof, or their solvate according to any one of [1] to [3] as an active ingredient;

[7] a use of the spiro compound or salt thereof, or their solvate according to any one of [1] to [3] for producing a formulation for inhibiting 11β-hydroxysteroid dehydrogenase 1;

[8] a use of the spiro compound or salt thereof, or their solvate according to any one of [1] to [3] for producing a formulation for an agent for preventing and/or treating diabetes, insulin resistance, diabetes complication, obesity, dyslipidemia, hypertension, fatty liver, or metabolic syndrome;

[9] a method for inhibiting 11β-hydroxysteroid dehydrogenase 1, which method comprises administering an effective amount of the spiro compound or salt thereof, or their solvate according to any one of [1] to [3];

[10] a method for preventing and/or treating diabetes, insulin resistance, diabetes complication, obesity, dyslipidemia, hypertension, fatty liver, or metabolic syndrome, which method comprises administering an effective amount of the spiro compound or salt thereof, or their solvate according to any one of [1] to [3].

### Effect of the Invention

A spiro compound of the present invention shows a superior inhibitory effect of 11β-hydroxysteroid dehydrogenase 1, and is useful as an agent for preventing or treating a disease that involves 11β-hydroxysteroid dehydrogenase 1 (in particular, diabetes, insulin resistance, diabetes complication, obesity, dyslipidemia, hypertension, fatty liver, or metabolic syndrome).

### Description of Embodiments

The present invention will be explained in detail herein below.

### (Compound of the present invention)

A "halogen atom" in the present invention can be specifically exemplified by a fluorine atom, chlorine atom, bromine atom and iodine atom.

In the present invention, a "C₁₋₆ alkyl group" means a straight-chained or branched-chained alkyl group with 1 to 6 carbons, and the examples include a methyl group, ethyl group, n-propyl group, isopropyl group, n-butyl group, isobutyl group, s-butyl group, t-butyl group, n-pentyl group, 2-methylbutyl group, 2,2-dimethylpropyl group, n-hexyl group, etc.

In the present invention, a "C₂₋₆ alkenyl group" means a straight-chained or branched-chained alkenyl group with 2 to 6 carbons, having a carbon-carbon double bond at any one or more sites on the alkyl chain. The examples include a vinyl group, prop-1-en-1-yl group, allyl group, isopropenyl group, but-1-en-1-yl group, but-2-en-1-yl group, but-3-en-1-yl group, 2-methylprop-2-en-1-yl group, 1-methylprop-2-en-1-yl group, pent-1-en-1-yl group, pent-2-en-1-yl group, pent-3-en-1-yl group, pent-4-en-1-yl group, 3-methylbut-2-en-1-yl group, 3-methylbut-3-en-1-yl group, hex-1-en-1-yl group, hex-2-en-1-yl group, hex-3-en-1-yl group, hex-4-en-1-yl group, hex-5-en-1-yl group, 4-methylpent-3-en-1-yl group, etc.

In the present invention, a "C₂₋₆ alkynyl group" means a straight-chained or branched-chained alkynyl group with 2 to 6 carbons, having a carbon-carbon triple bond at any one or more sites on the alkyl chain. The examples include an ethynyl group, prop-1-yn-1-yl group, prop-2-yn-1-yl group, but-1-yn-1-yl group, but-3-yn-1-yl group, 1-methylprop-2-yn-1-yl group, pent-1-yn-1-yl group, pent-4-yn-1-yl group, hex-1-yn-1-yl group, hex-5-yn-1-yl group, etc.

In the present invention, a "C₃₋₈ cycloalkyl group" means a cycloalkyl group with 3 to 8 carbons having a cyclic moiety. Examples include a cyclopropyl group, cyclobutyl group, cyclopentyl group, cyclohexyl group, cyclopropylmethyl group, cyclopropylethyl group, cyclobutylmethyl group, cyclopentylmethyl group, cyclohexylmethyl group, etc.

In the present invention, a "halo C₁₋₆ alkyl group" means a group wherein preferably 1 to 9 halogen atoms are bound to a C₁₋₆ alkyl group and the examples include trifluoromethyl group, 2-fluoroethyl group, 2-chloroethyl group, 2-bromoethyl group, 3-fluoropropyl group, 3-chloropropyl group, 4-fluorobutyl group, 4-chlorobutyl group, 2,2,2-trifluoroethyl group, 3,3,3-trifluoropropyl group, pentafluoroethyl group, 2,2,2-trifluoro-1-trifluoromethylethyl group, etc.

In the present invention, a "C₁₋₆ alkoxy group" refers to a group wherein the above "C₁₋₆ alkyl group" is bound to an oxygen atom, and the examples include a methoxy group, ethoxy group, n-propoxy group, isopropoxy group, n-butoxy group, isobutoxy group, s-butoxy group, t-butoxy group, n-pentoxy group, isopentoxy group, neopentoxy group, 1-methylbutoxy group, 1-ethylpropoxy group, n-hexyloxy group, isohexyloxy group, 4-methylpentoxy group, 3-methylpentoxy group, 2-methylpentoxy group, 1-methylpentoxy group, 3,3-dimethylbutoxy group, 2,2-dimethylbutoxy group, 1,1-dimethylbutoxy group, 1,2-dimethylbutoxy group, 1,3-dimethylbutoxy group, 2,3-dimethylbutoxy group, 1-ethylbutoxy group, 2-ethylbutoxy group, etc.

In the present invention, a "halo C₁₋₆ alkoxy group" means a group wherein the above halo C₁₋₆ alkyl group is bound to an oxygen atom, and the examples include a trifluoromethoxy group, 2-fluoroethoxy group, 2-chloroethoxy group, 2-bromoethoxy group, 3-fluoropropoxy group, 3-chloropropoxy group, 4-fluorobutoxy group, 4-chlorobutoxy group, 2,2,2-trifluoroethoxy group, 3,3,3-trifluoropropoxy group, pentafluoroethoxy group, 2,2,2-trifluoro-1-trifluoromethylethoxy group, etc.

In the present invention, a "C₁₋₆ alkoxy-C₁₋₆ alkyl group" means a group wherein the above "C₁₋₆ alkoxy group" is bound to a "C₁₋₆ alkyl group", and the examples include a methoxymethyl group, methoxyethyl group, ethoxymethyl group, ethoxyethyl group, etc.

In the present invention, a "C₆₋₁₀ aryl group" means a monocyclic or condensed ring aromatic hydrocarbon group with 6 to 10 carbon atoms. Herein, in case of a condensed ring, partial saturated groups are encompassed in addition to fully unsaturated groups. Examples include a phenyl group, naphthyl group, azulenyl group, indenyl group, indanyl group, tetralinyl group, etc.

In the present invention, a "C₆₋₁₀ aryloxy group" means a group wherein the above "C₆₋₁₀ aryl group" is bound to an oxygen atom, and the examples include a phenoxy group, naphtoxy group, azulenyloxy group, etc.

In the present invention, a "5- to 10-membered heteroaryl group" means a 5- to 6-membered monocyclic aromatic heterocyclic group containing 1 to 4 heteroatoms selected from a nitrogen atom, oxygen atom and sulfur atom in addition to a carbon atom as atoms constituting the ring; or a condensed aromatic heterocyclic group wherein these heterocycles and benzene are condensed. Examples of monocyclic aromatic heterocyclic group include a 2-furyl group, 3-furyl group, 2-thienyl group, 3-thienyl group, pyrrol-1-yl group, pyrrol-2-yl group, pyrrol-3-yl group, pyridin-2-yl group, pyridin-3-yl group, pyridin-4-yl group, pyrazin-2-yl group, pyrimidin-2-yl group, pyrimidin-4-yl group, pyrimidin-5-yl group, pyridazin-3-yl group, pyridazin-4-yl group, imidazol-1-yl group, imidazol-2-yl group, imidazol-4-yl group, pyrazol-1-yl group, pyrazol-3-yl group, pyrazol-4-yl group, thiazol-2-yl group, thiazol-3-yl group, thiazol-4-yl group, thiazol-5-yl group, oxazol-2-yl-group, oxazol-4-yl group, oxazol-5-yl group, isooxazol-3-yl group, isooxazol-4-yl group, isooxazol-5-yl group, 1,3,4-thiadiazol-2-yl group, 1,2,3-triazol-1-yl group, 1,2,3-triazol-4-yl group, 1,2,4-triazol-1-yl group, 1,2,4-triazol-3-yl group, 1,2,4-triazol-4-yl group, tetrazol-1-yl group, tetrazol-5-yl group, etc. Examples of condensed aromatic heterocyclic group include a benzofuran-2-yl group, benzofuran-3-yl group, benzofuran-4-yl group, benzofuran-5-yl group, benzofuran-6-yl group, benzofuran-7-yl group, benzothiophen-2-yl group, benzothiophen-3-yl group, benzothiophen-4-yl group, benzothiophen-5-yl group, benzothiophen-6-yl group, benzothiophen-7-yl group, quinoxalin-2-yl group, quinoxalin-5-yl group, quinoxalin-6-yl group, indol-1-yl group, indol-2-yl group, indol-3-yl group, indol-4-yl group, indol-5-yl group, indol-6-yl group, indol-7-yl group, isolndol-1-yl group, isoindol-2-yl group, isoindol-4-yl group, isoindol-5-yl group, isobenzofuran-1-yl group, isobenzofuran-4-yl group, isobenzofuran-5-yl group, chromen-2-yl group, chromen-3-yl group, chromen-4-yl group, chromen-5-yl group, chromen-6-yl group, chromen-7-yl group, chromen-8-yl group, benzoimidazol-1-yl group, benzoimidazol-2-yl group, benzoimidazol-4-yl group, benzoimidazol-5-yl group, benzothiazol-2-yl group, benzothiazol-4-yl group, benzothiazol-5-yl group, benzooxazol-2-yl group, benzooxazol-4-yl group, benzooxazol-5-yl group, quinolin-2-yl group, quinolin-3-yl group, quinolin-4-yl group, quinolin-5-yl group, quinolin-6-yl group, quinolin-7-yl group, quinolin-8-yl group, isoquinolin-1-yl group, isoquinolin-3-yl group, isoquinolin-4-yl group, isoquinolin-5-yl group, isoquinolin-6-yl group, isoquinolin-7-yl group, isoquinolin-8-yl group, etc.

In the present invention, examples of "C₁₋₆ alkylenedioxy group" include a methylenedioxy group, ethylenedioxy group, trimethylenedioxy group, tetramethylenedioxy group, pentamethylenedioxy group, hexamethylenedioxy group, etc.

In the present invention, a "C₁₋₆ alkanoyl group" means a group wherein an oxo group is bound to the 1st position of the above "C₁₋₆ alkyl group", and may be straight-chained or branched-chained. Therefore, examples of "C₁₋₆ alkanoyl group" include an acetyl group, propionyl group, butyryl group, isobutyryl group, valeryl group, isovaleryl group, pivaloyl group, etc.

In the present invention, a "mono C₁₋₆ alkylamino group" means a group wherein one of the above C₁₋₆ alkyl group is bound to the nitrogen atom of an amino group, and the examples include a methylamino group, ethylamino group, n-propylamino group, isopropylamino group, n-butylamino group, s-butylamino group, t-butylamino group, n-pentylamino group, isopentylamino group, neopentylamino group, 1-methylbutylamino group, 1-ethylpropylamino group, n-hexylamino group, isohexylamino group, 4-methylpentylamino group, 3-methylpentylamino group, 2-methylpentylamino group, 1-methylpentylamino group, 3,3-dimethylbutylamino group, 2,2-dimethylbutylamino group, 1,1-dimethylbutylamino group, 1,2-dimethylbutylamino group, 1,3-dimethylbutylamino group, 2,3-dimethylbutylamino group, 1-ethylbutylamino group, 2-ethylbutylamino group, etc.

In the present invention, a "di C₁₋₆ alkylamino group" means a group wherein 2 same or different above C₁₋₆ alkyl groups are bound to a nitrogen atom, and the examples include a dimethylamino group, methylethylamino group, diethylamino group, methyl-n-propylamino group, ethyl-n-propylamino group, di-n-propylamino group, methyl isopropylamino group, ethyl isopropylamino group, diisopropylamino group, methyl-n-butylamino group, ethyl-n-butylamino group, n-propyl-n-butylamino group, di-n-butylamino group, di-s-butylamino group, di-t-butylamino group, di-n-pentylamino group, di-n-hexylamino group, etc.

A "C₁₋₆ alkoxycarbonyl group" of the present invention means a group wherein the above "C₁₋₆ alkoxy group" is bound to a carbonyl group (C = O), and the examples include a methoxycarbonyl group, ethoxycarbonyl group, n-propoxycarbonyl group, isopropoxycarbonyl group, n-butoxycarbonyl group, isobutyloxycarbonyl group, s-butoxycarbonyl group, t-butoxycarbonyl group, n-pentoxycarbonyl group, isopentoxycarbonyl group, neopentoxycarbonyl group, 1-methylbutoxycarbonyl group, 1-ethylpropoxycarbonyl group, n-hexyloxycarbonyl group, isohexyloxycarbonyl group, 4-methylpentoxycarbonyl group, 3-methylpentoxycarbonyl group, 2-methylpentoxycarbonyl group, 1-methylpentoxycarbonyl group, 3,3-dimethylbutoxycarbonyl group, 2,2-dimethylbutoxycarbonyl group, 1,1-dimethylbutoxycarbonyl group, 1,2-dimethylbutoxycarbonyl group, 1,3-dimethylbutoxycarbonyl group, 2,3-dimethylbutoxycarbonyl group, 1-ethylbutoxycarbonyl group, 2-ethylbutoxycarbonyl group, etc.

In the present invention, a "C₁₋₆ alkylthio group" means a group wherein the above "C₁₋₆ alkyl group" is bound via a sulfur atom, and the examples include a methylthio group, ethylthio group, n-propylthio group, isopropylthio group, n-butylthio group, isobutylthio group, s-butylthio group, t-butylthio group, n-pentylthio group, isopentylthio group, neopentylthio group, 1-methylbutylthio group, 1-ethylpropylthio group, n-hexylthio group, isohexylthio group, 4-methylpentylthio group, 3-methylpentylthio group, 2-methylpentylthio group, 1-methylpentylthio group, 3,3-dimethylbutylthio group, 2,2-dimethylbutylthio group, 1,1-dimethylbutylthio group, 1,2-dimethylbutylthio group, 1,3-dimethylbutylthio group, 2,3-dimethylbutylthio group, 1-ethylbutylthio group, 2-ethylbutylthio group, etc.

In the present invention, a "C₆₋₁₀ arylthio group" means a group wherein the above "C₆₋₁₀ aryl group" is bound via a sulfur atom, and the examples include a phenylthio group, naphtylthio group, azulenylthio group, etc.

In the present invention, a "C₁₋₆ alkylsulfonyl group" means a group wherein the above "C₁₋₆ alkyl group" is bound via a sulfonyl group, and the examples include a methylsulfonyl group, ethylsulfonyl group, n-propylsulfonyl group, isopropylsulfonyl group, n-butylsulfonyl group, isobutylsulfonyl group, s-butylsulfonyl group, t-butylsulfonyl group, n-pentylsulfonyl group, isopentylsulfonyl group, neopentylsulfonyl group, 1-methylbutylsulfonyl group, 1-ethylpropylsulfonyl group, n-hexylsulfonyl group, isohexylsulfonyl group, 4-methylpentylsulfonyl group, 3-methylpentylsulfonyl group, 2-methylpentylsulfonyl group, 1-methylpentylsulfonyl group, 3,3-dimethylbutylsulfonyl group, 2,2-dimethylbutylsulfonyl group, 1,1-dimethylbutylsulfonyl group, 1,2-dimethylbutylsulfonyl group, 1,3-dimethylbutylsulfonyl group, 2,3-dimethylbutylsulfonyl group, 1-ethylbutylsulfonyl group, 2-ethylbutylsulfonyl group, etc.

In the present invention, a "C₆₋₁₀ arylsulfonyl group" means a group wherein the above "C₆₋₁₀ aryl group" is bound via a sulfonyl group, and the C₆₋₁₀ aryl group may be substituted with a C₁₋₆ alkyl group. Therefore, examples of "C₆₋₁₀ arylsulfonyl group" include a benzenesulfonyl group, toluenesulfonyl group, naphtalenesulfonyl group, etc.

Further, for groups which are not defined herein, the normal definition is applied.

In the present invention, preferred embodiment of general formula (1) is as follows.

In general formula (1), R¹ and R² are preferably a hydrogen atom.

In general formula (1), R³ and R⁴ are preferably a hydrogen atom.

In general formula (1), preferred examples of R⁵, R⁶, R⁷, R⁸ and R⁹ include a hydrogen atom, C₁₋₆ alkyl group, C₃₋₈ cycloalkyl group, halo C₁₋₆ alkyl group, and halogen atom. As C₁₋₆ alkyl group, a C₁₋₄ alkyl group is more preferred, and a methyl group, ethyl group, and n-propyl group are particularly preferred. As C₃₋₈ cycloalkyl group, a cyclopropyl group is more preferred. As halo C₁₋₆ alkyl group, a trifluoromethyl group is more preferred. As halogen atom, an iodine atom is more preferred. A combination wherein R⁵ is any one of C₁₋₆ alkyl group, C₃₋₈ cycloalkyl group, halo C₁₋₆ alkyl group and halogen atom, and R⁶, R⁷, R⁸ and R⁹ are a hydrogen atom is particularly preferred.

In general formula (1), R¹⁰ is preferably a hydrogen atom.

In general formula (2), a case where R¹¹, R¹², R¹³ and R¹⁴ are a hydrogen atom, hydroxy group, or C₁₋₆ alkyl group, and where R¹¹ and R¹², or R¹³ and R¹⁴ together form an oxygen atom is preferred. As C₁₋₆ alkyl group, a C₁₋₄ alkyl group is more preferred, and a methyl group is particularly preferred.

In general formula (3), R¹⁵ and R¹⁶ are preferably a hydrogen atom, C₁₋₆ alkyl group, C₃₋₈ cycloalkyl group and halogen atom. As C₁₋₆ alkyl group, a C₁₋₄ alkyl group is more preferred, and a methyl group, ethyl group, n-propyl group, and isopropyl group are more preferred. As C₃₋₈ cycloalkyl group, a cyclopropyl group is more preferred. As halogen atom, a chlorine atom and bromine atom are more preferred.

When an asymmetric carbon atom is present in the spiro compound shown by general formula (1) of the present invention, there exists an optical isomer, and the present invention encompasses those optical isomers or any mixtures comprising racemate and the like.

The present invention also encompasses various hydrates or solvates of the spiro compound shown by general formula (1) or pharmaceutically acceptable acid-addition salt thereof, and a crystal polymorphic substance of the same.

Examples of pharmaceutically acceptable salt of the spiro compound shown by general formula (1) specifically include acid-addition salt and the like treated with an inorganic acid (for example, a hydrochloric acid, hydrobromic acid, hydroiodic acid, sulfuric acid, nitric acid, phosphoric acid and the like) or an organic acid (for example, a formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, asparaginic acid, glutamic acid and the like).

Examples of solvates of the spiro compound shown by general formula (1) or pharmaceutically acceptable salt thereof include hydrates or various solvates (for example, a solvate with alcohol such as ethanol).

### (Preparation method)

### I. Method for producing a compound represented by (I), or salt thereof, or their solvates

Reacting cyclic amines shown by general formula (II) with carboxylic acids shown by general formula (III) or reactive derivatives thereof yields amide derivatives shown by general formula (IV). By allowing a phenol derivative shown by general formula (V) to react with the obtained compound shown by general formula (IV), a spiro compound (I) of interest can be produced.
The reaction path is shown by the following chemical formula.

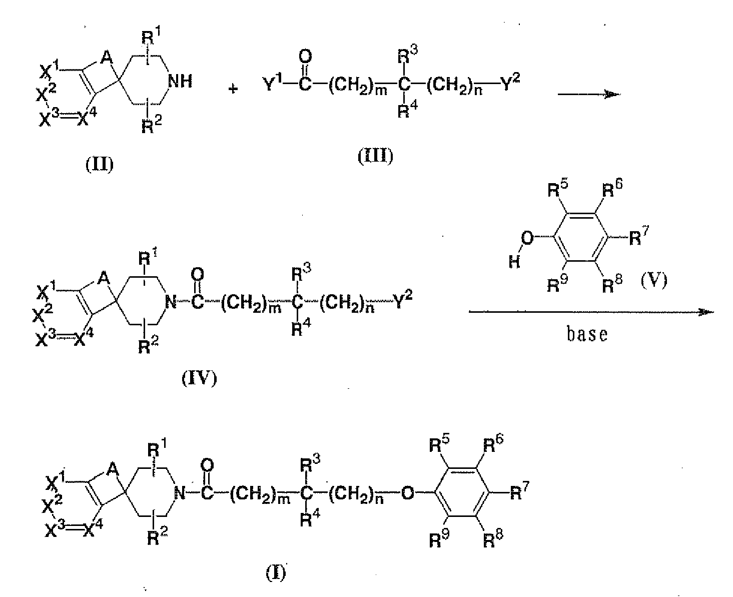

(wherein R¹, R², R³, R⁴, R⁵, R⁶, R⁷, R⁸, R⁹, X¹, X², X³, X⁴, A, m and n show the same things as they show in the above general formula (1), Y¹ shows a halogen atom, hydroxy group or aryloxy group, Y² shows a hydroxy group, halogen atom, alkylsulfonyloxy group, haloalkylsulfonyloxy group, or arylsulfonyloxy group).

A reaction of an amine compound (II) with an acid halide compound (III) wherein Y¹ is a halogen atom can be conducted in a solvent in the presence or absence of a base. A solvent is not particularly limited, and for example, the followings can be used independently or in combination: 1,2-dichloroethane, chloroform, dichloromethane, ethyl acetate, isopropyl acetate, toluene, benzene, tetrahydrofuran, dioxane, acetonitrile, propionitrile, N,N-dimethylformamide, N-methyl pyrrolidone and the like. A base is not particularly limited, and for example, the followings can be used: organic bases such as pyridine, N,N-dimethylaminopyridine (DMAP), collidine, lutidine, 1,8-diazabicyclo[5.4.0]undecene (DBU), 1,5-diazabicyclo[4,3,0]nonene (DBN), 1,4-diazabicyclo[2.2.2]octene (DABCO), triethylamine, 2,6-di-t-butylpyridine, N,N-diisopropylethylamine, N,N-diisopropylpentylamine, N-methylmorpholine, and trimethylamine; alkali metal hydrides such as lithium hydride, sodium hydride, and potassium hydride; alkali metal hydroxides such as lithium hydroxide, sodium hydroxide, and potassium hydroxide; alkali metal carbonates such as lithium carbonate, sodium carbonate, potassium carbonate, and cesium carbonate; bicarbonate metals such as sodium hydrogen carbonate and potassium hydrogen carbonate; lithium diisopropylamide, sodium diisopropylamide, potassium diisopropylamide, lithium hexamethyldisilazide, sodium hexamethyldisilazide, potassium hexamethyldisilazide, sodium t-butoxide, potassium t-butoxide, n-butyllithium, s-butyllithium, t-butyllithium and the like. The reaction condition varies depending on the materials used, but generally, an amide compound (IV) is obtained by conducting the reaction at -20 to 100°C, preferably at 0 to 30°C for 5 minutes to 2 days, preferably for 10 min to 24 hours.

A reaction of a compound (II) with a carboxylic acid compound (III) wherein Y¹ is a hydroxy group can be conducted in a solvent using a condensation agent in the presence or absence of a base, and in the presence or absence of a condensation accelerator. A solvent is not particularly limited, and for example, the followings can be used: 1,2-dichloroethane, chloroform, dichloromethane, ethyl acetate, isopropyl acetate, toluene, benzene, tetrahydrofuran, dioxane, acetonitrile, propionitrile, N,N-dimethylformamide, N-methylpyrrolidone and the like. A base is not particularly limited, and for example, the followings can be used: organic bases such as pyridine, DMAP, collidine, lutidine, DBU, DBN, DABCO, triethylamine, diisopropylethylamine, diisopropylpentylamine, trimethylamine and the like; alkali metal hydrides such as lithium hydride, sodium hydride, and potassium hydride; alkali metal hydroxides such as lithium hydroxide, sodium hydroxide, and potassium hydroxide; alkali metal carbonates such as lithium carbonate, sodium carbonate, potassium carbonate, and cesium carbonate; and bicarbonates such as sodium hydrogen carbonate and potassium hydrogen carbonate. A condensation accelerator is not particularly limited, and the followings can be used: DMAP, 1-hydroxy-7-azobenzotriazole (HOAt), 1-hydroxybenzotriazole (HOBt), 3-hydroxy-3,4-dihydro-4-oxo-1,2,3-benzotriazole (HODhbt), N-hydroxy-5-norbornene-2,3-dicarboxyimide (HONB), pentafluorophenol (HOPfp), N-hydroxyphthalimide (HOPht), N-hydroxysuccinimide (HOSu) and the like. A condensation agent is not particularly limited, and the followings can be used: N,N'-dicyclohexylcarbodiimide (DCC), N,N'-diisopropylcarbodiimide (DIPCI), 1-ethyl-3-(3-dimethylaminopropyl)carbodiimide (WSCI), 1-ethyl-3-(3-dimethylaminopropyl)carbodiimide hydrochloride (WSC·HCl), diethyl cyanophosphate (DEPC), benzotriazol-1-yl-oxy-tris(dimethylamino)phosphoniumhexa fluorophosphate (BOP), benzotriazol-1-yl-oxy-tris(pyrrolidinylamino)phosphoniumhex a fluorophosphate (PyBOP), 2-(1H-benzotriazol-1-yl)-1,1,3,3-tetramethyluronium tetrafluoroborate (TBTU) and the like. The reaction condition varies depending on the materials used, but generally, an amide compound (IV) is obtained by conducting the reaction at -20 to 100° C, preferably at 0 to 30° C for 5 minutes to 2 days, preferably for 2 to 24 hours.

When Y² of the obtained amide compound (IV) is a halogen atom, alkylsulfonyloxy group, haloalkylsulfonyloxy group, or arylsulfonyloxy group, the reaction of a compound (IV) with a phenol derivative (V) can be conducted in a solvent in the presence of a base. A solvent is not particularly limited, and for example, the followings can be used independently or in combination: tetrahydrofuran, toluene, dioxane, N,N-dimethylformamide, N-methylpyrrolidone, dichloromethane, acetonitrile, propionitrile and the like. A base is not particularly limited, and for example, the followings can be used: alkali metal hydrides such as lithium hydride, sodium hydride, and potassium hydride; alkali metals such as lithium metal, sodium metal, and potassium metal; alkali metal hydroxides such as lithium hydroxide, sodium hydroxide, and potassium hydroxide; alkali metal carbonates such as lithium carbonate, sodium carbonate, potassium carbonate, and cesium carbonate; DABCO, lithium diisopropylamide, sodium diisopropylamide, potassium diisopropylamide, lithium hexamethyldisilazide, sodium hexamethyldisilazide, potassium hexamethyldisilazide, sodium t-butoxide, potassium t-butoxide, n-butyllithium, s-butyllithium, t-butyllithium and the like. The reaction condition varies depending on the materials used, but generally, a spiro compound (I) of interest is obtained by conducting the reaction at -20 to 150°C, preferably at 15 to 80°C for 5 minutes to 3 days, preferably for 5 to 50 hours.

When Y² of the obtained amide compound (IV) is a hydroxy group, the compound (IV) and a phenol derivative (V) can be subjected to the Mitsunobu reaction. The Misunobu reaction may be conducted by using a phosphine reagent and an azo reagent or ethylenedicarboxylic acid reagent, or by using a phosphonium ylide reagent. Examples of a preferred embodiment of this process include 1) a method of reacting a phenol derivative (V) or salt thereof in the presence of a phosphine reagent and azo reagent or ethylenedicarboxylic acid reagent such as dimethyl maleate, N,N,N',N'-tetramethylfumaramide and the like (the first method), and 2) a method of reacting a phenol derivative (V) in the presence of a phosphonium ylide reagent (the second method).

The first method can be conducted by dissolving an amide compound (IV), phenol derivative (V), and a phosphine reagent in a reaction solvent, and adding thereto an azo reagent or ethylendicarboxylic acid reagent, and performing a reaction under an argon or nitrogen atmosphere at 0°C to 100° C, preferably at room temperature to 80°C for 2 hours to 1 day. As a solvent to be used in this reaction, the followings can be used: N,N-dimethylformamide, tetrahydrofuran, dioxane, acetonitrile, nitromethane, acetone, ethyl acetate, benzene, chlorobenzene, toluene, chloroform, dichloromethane, and the like. Among these, toluene, tetrahydrofuran, dioxane, acetonitrile, and dichloromethane are preferred, and toluene and tetrahydrofuran are particularly preferred. Examples of a phosphine reagent include, for example, trialkylphosphines such as trimethylphosphine, triethylphosphine, tripropylphosphine, triisopropylphosphine, tributylphosphine, triisobutylphosphine, tricyclohexylphosphine and the like; and arylphosphines such as triphenylphosphine and diphenylphosphino polystyrene. Among these, trimethylphosphine, tributylphosphine, and triphenylphosphine are preferred. Examples of an azo reagent include, for example, diethyl azodicarboxylate (DEAD), diisopropyl azodicarboxylate, 1,1'-azobis(N,N-methylformamide) (TMAD), 1,1'-(azodicarbonyl)dipiperidine (ADDP), 1,1'-azobis(N,N-diisopropylformamide) (TIPA), 1,6-dimethyl-1,5,7-hexahydro-1,4,6,7-tetrazocine-2,5-dione (DHTD) and the like, and DEAD is particularly preferred.

The second method can be conducted by dissolving an amide compound (IV), phenol derivative (V), and a phosphonium ylide reagent in a reaction solvent, and performing a reaction under an argon or nitrogen atmosphere at room temperature to 120°C, preferably at 80°C to 100°C for 2 hours to 12 hours.
Examples of a phosphonium ylide reagent used in this reaction include alkanoylmethylene trialkylphosphorane, alkanoylmethylene triarylphosphorane, alkoxycarbonylmethylene trialkylphosphorane, alkoxycarbonylmethylene triarylphosphorane, cyanomethylene trialkylphosphorane, cyanomethylene triarylphosphorane and the like. Here, examples of trialkyl include trimethyl, triethyl, tripropyl, triisopropyl, tributyl, triisobutyl, and tricyclohexyl, and examples of triaryl include triphenyl and diphenyl polystyrene. Further, the present reaction may be conducted using a method comprising allowing a phosphonium halide reagent to act on an amide compound (IV), phenol derivative (V) or salt thereof in the presence of a base to produce a phosphonium ylide reagent in the reaction system.
Examples of a phosphonium halide reagent used in this case include, for example, (cyanomethyl)trialkylphosphonium halide, (cyanomethyl)triarylphosphonium halide, (alkylcarbonylmethyl)trialkylphosphonium halide, (alkylcarbonylmethyl)triarylphosphonium halide, (alkoxycarbonylmethyl)trialkylphosphonium halide, and (alkoxycarbonylmethyl)triarylphosphonium halide.

Further, a compound shown by formula (I) can also be produced according to the reaction formula below.

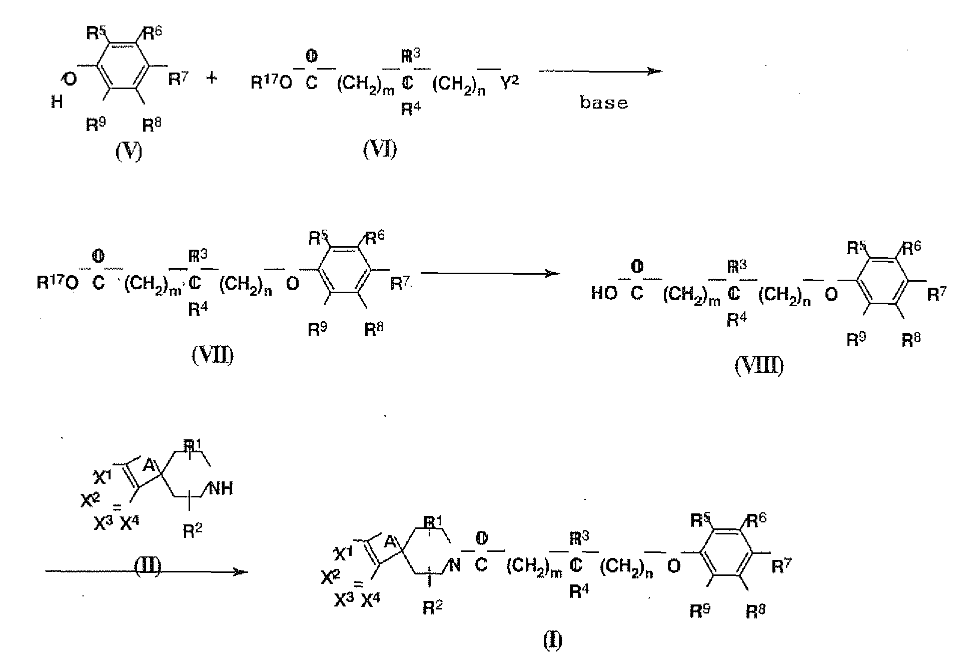

(wherein R¹, R², R³, R⁴, R⁵, R⁶, R⁷, R⁸, R⁹, X¹, X², X³, X⁴, A, m and n show the same things as they show in the above general formula (1), R¹⁷ shows a lower-alkyl group, and Y² shows a halogen atom, alkylsulfonyloxy group, haloalkylsulfonyloxy group, or arylsulfonyloxy group).

A reaction of a phenol derivative (V) with a carboxylate ester derivative (VI) having a leaving group can be conducted in a solvent in the presence of a base. A solvent is not particularly limited, and for example, the followings can be used independently or in combination: tetrahydrofuran, toluene, dioxane, N,N-dimethylformamide, N-methylpyrrolidone, dichloromethane, acetonitrile, propionitrile and the like. A base is not particularly limited, and for example, the followings can be used: alkali metal hydrides such as lithium hydride, sodium hydride, and potassium hydride; alkali metals such as lithium metal, sodium metal, and potassium metal; alkali metal hydroxides such as lithium hydroxide, sodium hydroxide, and potassium hydroxide; alkali metal carbonates such as lithium carbonate, sodium carbonate, potassium carbonate, and cesium carbonate; DABCO, lithium diisopropylamide, sodium diisopropylamide, potassium diisopropylamide, lithium hexamethyldisilazide, sodium hexamethyldisilazide, potassium hexamethyldisilazide, sodium t-butoxide, potassium t-butoxide, n-butyllithium, s-butyllithium, t-butyllithium and the like. The reaction condition varies depending on the materials used, but generally, a substance of interest (VII) is obtained by conducting the reaction at -20 to 150°C, preferably at 15 to 80°C for 5 minutes to 1 day, preferably for 5 to 12 hours.

The obtained carboxylate ester derivative (VII) is subjected to a hydrolysis reaction to yield a carboxylic acid form (VIII). The present reaction can be conducted in a solvent in the presence of abase or acid. A solvent is not particularly limited, and for example, the followings can be used independently or in combination: tetrahydrofuran, dioxane, methanol, ethanol, water, chlorobenzene, 1,2-dichloroethane, dichloromethane, chloroform, nitrobenzene, nitromethane and the like. A base is not particularly limited, and for example, the followings can be used: alkali metal hydroxides such as sodium hydroxide and potassium hydroxide; alkali metal carbonates such as lithium carbonate, sodium carbonate, potassium carbonate, and cesium carbonate; trimethylsilyloxy potassium and the like. An acid is not particularly limited, and the followings can be used: hydrochloric acid, acetic acid, trifluoroacetic acid, boron tribromide, aluminium chloride and the like. The reaction condition varies depending on the materials used, but generally, a carboxylic acid derivative (VIII) is obtained by conducting the reaction at -20 to 100° C, preferably at 0 to 50° C for 5 minutes to 2 days, preferably for 5 hours to 24 hours.

A condensation reaction of the obtained carboxylic acid derivative (VIII) with a compound (II) can be conducted using a condensation agent in the presence or absence of a base, and in the presence or absence of a condensation accelerator. A solvent is not particularly limited, and for example, the followings can be used: 1,2-dichloroethane, chloroform, dichloromethane, ethyl acetate, isopropyl acetate, toluene, benzene, tetrahydrofuran, dioxane, acetonitrile, propionitrile, N,N-dimethylformamide, N-methylpyrrolidone and the like. A base is not particularly limited, and for example, the followings can be used: organic bases such as pyridine, DMAP. collidine, lutidine, DBU, DBN, DABCO, triethylamine, diisopropylethylamine, diisopropylpentylamine, and trimethylamine; alkali metal hydrides such as lithium hydride, sodium hydride, and potassium hydride; alkali metal hydroxides such as lithium hydroxide, sodium hydroxide, and potassium hydroxide; alkali metal carbonates such as lithium carbonate, sodium carbonate, potassium carbonate, and cesium carbonate; and bicarbonates such as sodium hydrogen carbonate and potassium hydrogen carbonate. A condensation accelerator is not particularly limited, and DMAP, HOAt, HOBt, HODhbt, HONB, HOPfp, HOPht, HOSu and the like can be used. A condensation agent is not particularly limited, and DCC , DIPCI, WSCI, WSC·HCl, DEPC, BOP, PyBOP, TBTU and the like can be used. The reaction condition varies depending on the materials used, but generally, a spiro compound (I) of interest is obtained by conducting the reaction at -20 to 100° C, preferably at 0 to 30°C for 5 minutes to 1 day, preferably for 2 to 12 hours.

### II. Method for producing a compound represented by (II) or salt thereof, or their solvate

The spiro compound (II) used in the above-mentioned preparation method can be produced according to methods known from literatures, or pursuant to those methods, for example, according to the chemical formula below.

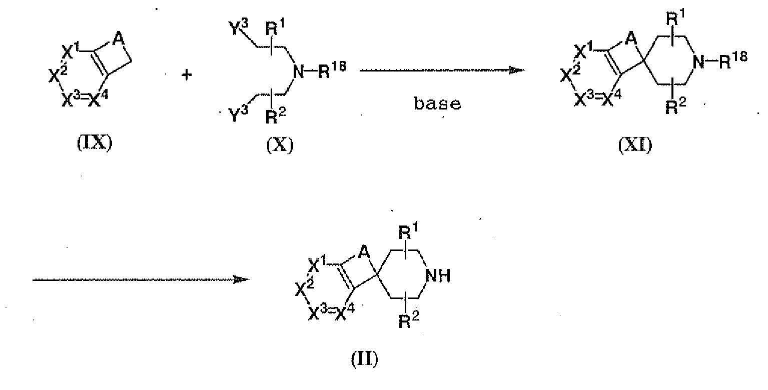

(wherein R¹, R², X¹, X², X³, X⁴ and A show the same things as they show in the above general formula (1), R¹⁸ shows a protecting group, and Y³ shows a halogen atom, alkylsulfonyloxy group, haloalkylsulfonyloxy group, or arylsulfonyloxy group).

A reaction of a cyclic compound (IX) with amine compound (X) can be conducted in a solvent in the presence of a base. A solvent is not particularly limited, and for example, the followings can be used independently or in combination: tetrahydrofuran, toluene, dioxane, N,N-dimethylformamide, dimethylsulfoxide (DMSO), hexane, acetonitrile, propionitrile and the like. A base is not particularly limited, and for example, the followings can be used: alkali metal hydrides such as lithium hydride, sodium hydride, and potassium hydride; alkali metal carbonates such as lithium carbonate, sodium carbonate, potassium carbonate, and cesium carbonate; DABCO, lithium diisopropylamide, sodium diisopropylamide, potassium diisopropylamide, lithium hexamethyldisilazide, sodium hexamethyldisilazide, potassium hexamethyldisilazide, sodium t-butoxide, potassium t-butoxide, n-butyllithium, s-butyllithium, t-butyllithium and the like. The reaction condition varies depending on the materials used, but generally, a spiro form (XI) is obtained by conducting the reaction at -20 to 150°C, preferably at 0 to 60° C for 5 minutes to 1 day, preferably for 10 min to 12 hours.

A deprotection of protective group R¹⁸ of the compound (XI) obtained in the above method is not particularly limited, which can be conducted with reference to a commonly used method (Protective Groups in Organic Synthesis Fourth Edition, John Wiley & Sons, Inc.) as a deprotection condition of the protective group. A protective group is not particularly limited, and for example, the followings can be used: a benzyl group, 9-fluorenylmethyloxycarbonyl group (Fmoc group), 2,2,2-trichloroethyloxycarbonyl group (Troc group), 2-trimethylsilylethyloxycarbonyl group (Teoc group), t-butoxycarbonyl group (Boc group), allyloxycarbonyl group (Alloc group), vinyloxycarbonyl group, benzyloxycarbonyl group (Cbz group), p-methoxybenzyloxycarbonyl group, p-nitrobenzyloxycarbonyl group, allyl group, 2-(trimethylsilyl)ethoxymethyl group (SEM group), 4-methoxybenzyl group, triphenylmethyl group, benzenesulfonyl group, and o-nitrobenzenesulfonyl group. In particular, a benzyl group, Fmoc group, Boc group, and Cbz group are preferred.

### III. Method for producing a compound represented by (IX) or salt thereof, or their solvate

As a cyclic compound (IX) used in the above preparation method, a commercially available compound can be used directly, or can be produced appropriately by a known method, or according to the same. For example, a cyclic compound (IX) can be produced by the following method depending on the type of R¹⁹ substituted on the 2nd position of an indene derivative (XIV), but it is not limited to this method.

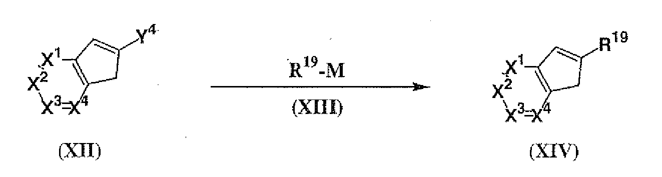

(wherein X¹, X², X³ and X⁴ show the same things as they show in the above general formula (1), R¹⁹ shows a C₁₋₆ alkyl group, halo C₁₋₆ alkyl group, C₂₋₆ alkenyl group, C₂₋₆ alkynyl group, C₃₋₈ cycloalkyl group, and C₆₋₁₀ aryl group; Y⁴ shows a halogen atom, alkylsulfonyloxy group, haloalkylsulfonyloxy group, or arylsulfonyloxy group).

For a reaction of an indene derivative (XII) and alkyl metal (XIII), a reaction technique conducted in a solvent in the presence or absence of a base, and in the presence of a metal catalyst can be applied. Metal M of general formula (XIII) is preferably a lithium, sodium, potassium, boron, tin, aluminum, magnesium, zinc, silicon, etc. A solvent is not particularly limited, and for example, the followings can be used independently or in combination: tetrahydrofuran, toluene, dioxane, N,N-dimethylformamide, N-methylpyrolidone, water, and the like. A base is not particularly limited, and for example, the followings can be used: alkali metal hydrides such as lithium hydride, sodium hydride, and potassium hydride; alkali metals such as lithium metal, sodium metal, and potassium metal; alkali metal hydroxides such as lithium hydroxide, sodium hydroxide, and potassium hydroxide; alkali metal carbonates such as lithium carbonate, sodium carbonate, potassium carbonate, and cesium carbonate; lithium diisopropylamide, sodium diisopropylamide, potassium diisopropylamide, lithium hexamethyldisilazide, sodium hexamethyldisilazide, potassium hexamethyldisilazide, sodium t-butoxide, potassium t-butoxide, n-butyllithium, s-butyllithium, t-butyllithium and the like. As a metal catalyst, for example, tris(dibenzylideneacetone)dipalladium (0), tris(dibenzylideneacetone) (chloroform) dipalladium (0), [1,1'-bis(diphenylphosphino)ferrocene]dichloropalladium (II), tetrakis(triphenylphosphine)palladium, etc. can be used independently, or by combining with ligands such as (2-biphenyl)di-t-butylphosphine or (2-biphenyl)dicyclohexylphosphine. The reaction condition varies depending on the materials used, but generally, a substance of interest is obtained by conducting the reaction at -20 to 180°C, preferably at 0 to 60°C for 5 minutes to 72 hours, preferably for 10 min to 24 hours.

### IV. Method for producing a compound represented by (XVI) or salt thereof, or their solvate

Among the carboxylic acid form (VIII) used in the above preparation method, for example, the compound shown by the general formula (XVI) can be produced according to the following reaction formula, other than the above mentioned method, but is not limited to this method.

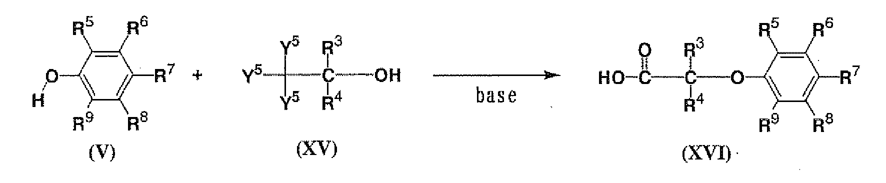

(wherein R³, R⁴ R⁵, R⁶, R⁷, R⁸ and R⁹ show the same things as they show in the above general formula (1), Y⁵ shows a halogen atom such as chlorine atom, bromine atom, and iodine atom).

A reaction of a phenol derivative (V) with alcohol form (XV) can be conducted in a solvent, in the presence of a base. A solvent is not particularly limited, and for example, the followings can be used independently or in combination: dioxane, methanol, ethanol, water, acetone, tetrahydrofuran, toluene, dioxane, N, N-dimethylformadide, N-methylpyrolidone, dichloromethane, acetonitrile, propionitrile and the like. A base is not particularly limited, and for example, the followings can be used: alkali metal hydrides such as lithium hydride, sodium hydride, and potassium hydride; alkali metals such as lithium metal, sodium metal, and potassium metal; alkali metal hydroxides such as lithium hydroxide, sodium hydroxide, and potassium hydroxide; alkali metal carbonates such as lithium carbonate, sodium carbonate, potassium carbonate, and cesium carbonate; DABCO, lithium diisopropylamide, sodium diisopropylamide, potassium diisopropylamide, lithium hexamethyldisilazide, sodium hexamethyldisilazide, potassium hexamethyldisilazide, sodium t-butoxide, potassium t-butoxide, n-butyllithium, s-butyllithium, t-butyllithium and the like. The reaction condition varies depending on the materials used, but generally, a substance of interest (XVI) is obtained by conducting the reaction at -20 to 150° C, preferably at 0 to 80° C for 5 minutes to 2 days, preferably for 5 hours to 24 hours.

### V. Method for producing a compound represented by (XVIII) or salt thereof, or their solvate

Among the compounds shown by general formula (I), for example, a compound shown by general formula (XVIII) can be produced according to the following reaction formula, but is not limited to this formula.

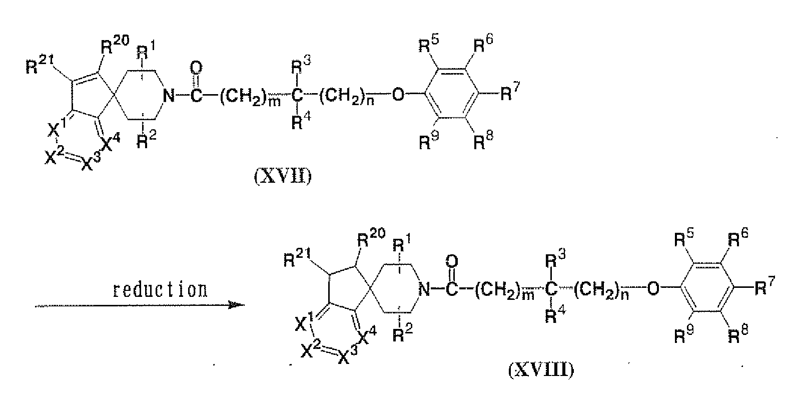

(wherein R¹, R², R³, R⁴, R⁵, R⁶, R⁷, R⁸, R⁹, X¹, X², X³, X⁴, m and n show the same things as they show in the above general formula (1) ; R²⁰ and R²¹ are the same or different and show a hydrogen atom, hydroxy group, halogen atom, C₁₋₆ alkyl group, C₁₋₆ alkoxy group, halo C₁₋₆ alkyl group, C₂₋₆ alkenyl group, C₂₋₆ alkynyl group, C₃₋₈ cycloalkyl group, or C₆₋₁₀ aryl group) .

As a reduction reaction of a compound shown by general formula (XVII), an usual method for converting an alkenyl group to alkyl group can be applied, and for example, a catalytic reduction using a metal catalyst and hydrogen source can be used. A hydrogen source for hydrogenation is not particularly limited, and hydrogen, formic acid, ammonium formate, cyclohexadiene and the like can be used. A hydrogenation catalyst is not particularly limited, and the followings can be used: palladium carbon, palladium black, platinum black, platinum dioxide, Raney nickel, palladium carbon hydroxide powder and the like. A solvent is not particularly limited, and for example, the followings can be used independently or in combination: methanol, ethanol, isopropanol, ethyl acetate, isopropyl acetate, N,N-dimethylformamide, tetrahydrofuran, dioxane, acetonitrile, acetic acid and water. The reaction condition varies depending on the compound used shown by general formula (XVII), but generally, a substance of interest is obtained by conducting the reaction at -20 to 150° C, preferably at 0 to 100°C for 5 minutes to 3 days, preferably for 30 min to 50 hours.

### VI. Method for producing a compound represented by (XXII) or salt thereof, or their solvate

Among the compounds shown by general formula (I), for example, a compound shown by general formula (XXII) can be produced according to the following reaction formula, but is not limited to this formula.

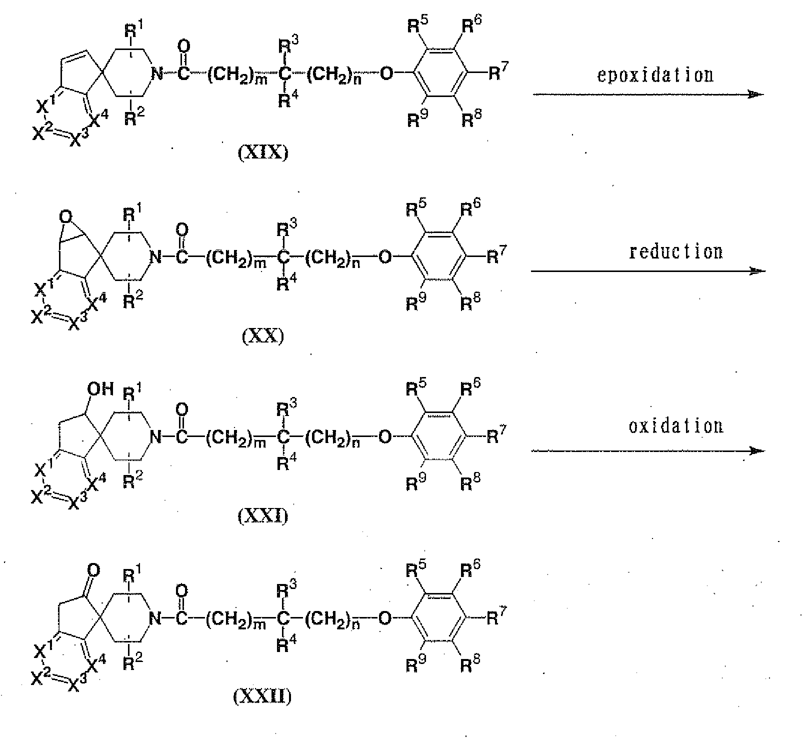

(wherein R¹, R², R³, R⁴, R⁵, R⁶, R⁷, R⁸, R⁹, X¹, X**²**, X³, X⁴, m and n show the same things as they show in the above general formula (1))

As an oxidation reaction of a compound shown by general formula (XIX) to a compound shown by general formula (XX), an usual method for oxidizing an alkenyl group to epoxy group can be used, and for example, a peroxide such as meta-chloroperbenzoic acid and peracetic acid, or a epoxylated reagent used by combining a metal reagent such as methylrhenium trioxide with a peroxide such as hydrogen peroxide can be used. At that time, pyridine and the like can be used as an additive. A solvent is not particularly limited, and the followings can be used independently or in combination: tetrahydrofuran, dichloromethane, chloroform, 1,2-dichloroethane, N,N-dimethylformamide and the like. The reaction condition varies depending on the compound used shown by general formula (XIX), but generally, a substance of interest is obtained by conducting the reaction at -20 to 150° C, preferably at 0 to 100° C for 5 minutes to 3 days, preferably for 30 minutes to 50 hours.

As a reduction reaction of a compound shown by general formula (XX) to a compound shown by general formula (XXI), an usual method for hydrolyzing benzylic ether can be applied, and for example, a catalytic reduction using a metal catalyst and hydrogen source can be used. A hydrogen source for hydrogenation is not particularly limited, and hydrogen, formic acid, ammonium formate, cyclohexadiene and the like can be used. A hydrogenation catalyst is not particularly limited, and the followings can be used: palladium carbon, palladium black, platinum black, platinum dioxide, Raney nickel, palladium carbon hydroxide powder and the like. A solvent is not particularly limited, and for example, the followings can be used independently or in combination: methanol, ethanol, isopropanol, ethyl acetate, isopropyl acetate, N,N-dimethylformamide, tetrahydrofuran, dioxane, acetonitrile, acetic acid, water, etc. The reaction condition varies depending on the compound used shown by general formula (XX), but generally, a substance of interest is obtained by conducting the reaction at -20 to 150° C, preferably at 0 to 100° C for 5 minutes to 3 days, preferably for 30 min to 50 hours.

As an oxidation reaction of a compound shown by general formula (XXI) to a compound shown by general formula (XXII), an usual method for oxidizing a hydroxy group to ketone can be applied, and for example, the oxidizing conditions of Swern oxidation, Moffatt oxidation, Dess-Martin oxidation; or pyridinium chlorochromate (PCC), pyridinium dichromate (PDC), manganese dioxide, tetrapropylammonium perruthenate (TPAP) and the like can be used. A solvent is not particularly limited, and the followings can be used independently or in combination: tetrahydrofuran, dichloromethane, chloroform, 1,2-dichloroethane, N,N-dimethylformamide and the like. The reaction condition varies depending on the compound used shown by general formula (XXI), but generally, a substance of interest is obtained by conducting the reaction at -20 to 150°C, preferably at 0 to 100°C for 5 minutes to 3 days, preferably for 30 minutes to 50 hours.

### VII. Method for producing a compound represented by (XXVI) or salt thereof, or their solvate

Among the compounds shown by general formula (II), for example, a compound shown by general formula (XXVI) can be produced according to the following reaction formula, but is not limited to this formula.

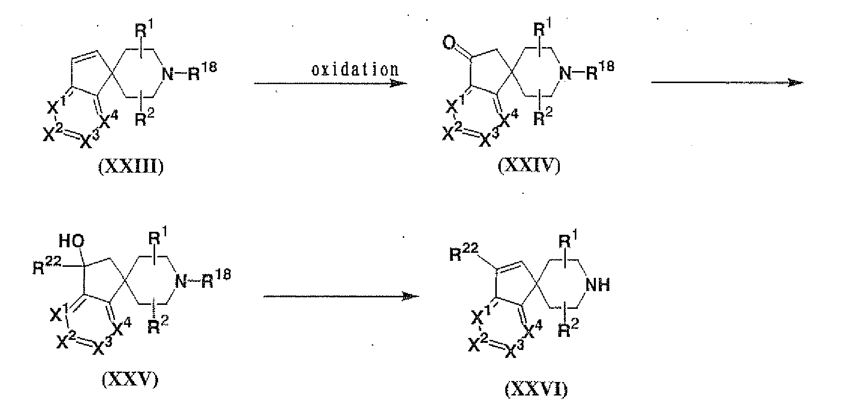

(wherein R¹, R², X¹, X², X³ and X⁴ show the same things as they show in the above general formula (1), R¹⁸ shows a protection group, and R²² shows a C₁₋₆ alkyl group, halo C₁₋₆ alkyl group, C₂₋₆ alkenyl group, C₂₋₆ alkynyl group, C₃₋₈ cycloalkyl group, or C₆₋₁₀ aryl group).

As an oxidation reaction of a compound shown by general formula (XXIII) to a compound shown by general formula (XXIV), an usual method for oxidizing an alkenyl group to ketone can be applied, and for example, a method combining hydroboration and oxidation, and the like can be used. As a hydroboration reagent, the followings can be used: 9-borabicyclo [3.3.1]nonane (9-BBN), disiamylborane (Sia₂BH), thexylborane (ThxBH₂), dicyclohexylborane (Cy₂BH), cathecholborane, borane-tetrahydrofuran complex, borane-dimethylsulphide complex, and the like. As an oxidation reagent, oxidants such as pyridinium chlorochromate (PCC), PDC, TPAP, sodium periodate and the like can be used. A solvent is not particularly limited, and the followings can be used independently or in combination: tetrahydrofuran, dichloromethane, chloroform, 1,2-dichloroethane, N,N-dimethylformamide and the like. The reaction condition varies depending on the compound used shown by general formula (XXIII), but generally, a substance of interest is obtained by conducting the reaction at -20 to 150° C, preferably at 0 to 100°C for 5 minutes to 3 days, preferably for 30 minutes to 50 hours.

As a reaction from a compound shown by general formula (XXIV) to a compound shown by general formula (XXV), a reaction technique conducted in a solvent by using a metal reagent can be applied. Preferred metals of a metal reagent used include a lithium, sodium, potassium, boron, tin, aluminum, magnesium, zinc, silicon, etc. A solvent is not particularly limited, and for example; the followings can be used independently or in combination: tetrahydrofuran, diethylether, toluene, dioxane, N,N-dimethylformamide, N-methylpyrolidone, water, and the like. The reaction condition varies depending on the materials used, but generally, a substance of interest is obtained by conducting the reaction at -100 to 180° C, preferably at -78 to 60°C for 5 minutes to 72 hours, preferably for 10 min to 24 hours.

As a reaction from a compound shown by general formula (XXV) to a compound shown by general formula (XXVI), a method combining a dehydration reaction and a method commonly used as a deprotection condition of the protection group (Protective Groups in Organic Synthesis Fourth Edition, John Wiley & Sons, Inc.) can be used. As a dehydration reaction, a dehydration reaction under for example acid conditions can be used. Acid is not particularly limited, and examples include proton acids such as acetic acid, trifluoroacetic acid, propionic acid, benzoic acid; and Lewis acid such as titanium tetrachloride, trifluoroborane, stannic chloride can be used. A solvent is not particularly limited, and the followings can be used independently or in combination: 1,2-dichloroethane, chloroform, dichloromethane, ethylacetate, isopropyl acetate, toluene, benzene, tetrahydrofuran, dioxane, acetonitrile, propionitrile, methanol, ethanol, isopropanol and the like. The reaction condition varies depending on the materials used, but generally, a substance of interest is obtained by conducting the reaction at -20 to 180°C, preferably at 0 to 60°C for 5 minutes to 72 hours, preferably for 10 min to 24 hours.

The intermediates and substances of interest obtained in each of the above reactions can be isolated and purified as desired by subjecting to a purification method that are used routinely in the field of organic synthetic chemistry, for example, filtration, extraction, washing, drying, condensation, recrystallization, various types of chromatography and the like. Alternatively, the intermediates can be used for next reactions without a particular purification.

Further, various isomers can be isolated by applying a routine procedure utilizing the difference in physical-chemical property between the isomers. For example, a racemic mixture can be led to optically-pure isomers by a common racemic resolution method such as an optical resolution method comprising leading a mixture to diastereomeric salt with a common optically-active acid such as tartaric acid, or a method using optioally-active column chromatography. Further, a diastereomeric mixture can be separated by a fractional crystallization, various types of chromatography or the like. Alternatively, an optically-active compound can be produced by using an appropriate optically-active material.

The pharmaceutical composition of the present invention comprises a spiro compound shown by general formula (1), pharmaceutically acceptable salt thereof, or their solvate as an active ingredient. The compound of the present invention can be used independently, but generally, the compound is used in combination with a pharmaceutically acceptable carrier and/or diluent.

Examples of an administration form of a medicine that comprises the compound of the present invention or pharmaceutically acceptable salt thereof, or their solvate as an active ingredient include an oral administration by a tablet, capsule, granules, powder, syrup or the like; or a parenteral administration by an intravenous injection, intramuscular injection, suppository, inhaler, percutaneous absorption, eye-drops, nasal preparation or the like. Further, to prepare a pharmaceutical formulation in such various forms, the active ingredient can be prepared independently or as a pharmaceutical composition where appropriate, by combining with other pharmaceutically acceptable carriers, specifically an excipient, binder, extender, disintegrant, surfactant, lubricant, dispersant, buffer, preservative, flavoring agent, flavor, coating agent, diluent or the like.

The dose of the medicine of the present invention varies depending on weight, age, sex, symptoms and the like of the patient, but generally, in a case of an adult, the compound represented by general formula (I) can be administered in an amount of 0.1 to 1000 mg, in particular 1 to 300 mg a day, as a single or several separate doses either orally or parenterally.

The present invention will be further described with reference to the following examples, while the scope of the present invention will not be limited to these examples.

### Example 1

### Preparation of 1-[spiro(indene-1,4'-piperidine)-1'-yl]-2-[2-(trifluorometh yl)phenoxy]ethanone

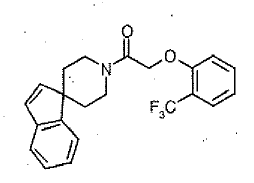

### Process 1: Preparation of t-butyl spiro(indene-1,4'-piperidine)-1'-carboxylate

t-Butyl spiro(indene-1,4'-piperidine)-1'-carboxylate wars produced by the method described below.

Under an argon atmosphere, a solution of indene (2.00 g, 18.0 mmol) in tetrahydrofuran (15 mL) was added with a solution of lithium hexamethyldisilazide in hexane (1.0 M, 36 mL) at 0°C. The resultant was stirred at the same temperature for 1 hour. Then, a solution of N-t-butyloxycarbonylbis(2-chloroethyl)amine (4.24 g, 18.0 mmol) in tetrahydrofuran (10 mL) was added thereto, and the mixture was further stirred at the same temperature for 2 hours. The reaction solution was concentrated in vacuo, and the resultant residue was purified by silica-gel chromatography (diethylether) and t-butyl spiro(indene-1,4'-piperidine)-1'-carboxylate (2.58 g, 50.2%) was obtained as a yellow amorphous solid.

¹H-NMR (400 MHz, CDCl₃) δ; 1.33 (d, J= 12.7 Hz, 2H), 1.51 (s, 9H), 1,96-2.07 (m, 2H), 3.07-3.17 (m, 2H), 4.10-4.21 (m, 2H), 6.78 (d, J= 5.7 Hz, 1H), 6.84 (d, J= 5.7 Hz, 1H), 7.20-7.34 (m, 4H).

### Process 2: Preparation of spiro(indene-1,4'-piperidine)

Spiro(indene-1,4'-piperidine) was produced by the method described below.

A solution of t-butyl spiro(indene-1,4'-piperidine)-1'-carboxylate (380 mg, 1.33 mmol) in dichloromethane(1 mL) was added with trifluoroacetic acid (0. 5 mL), and stirred at room temperature for 10 minutes. The reaction solution was concentrated in vacuo, and the resultant residue was diluted into chloroform. The resultant was washed with a saturated aqueous solution of sodium hydrogen carbonate, concentrated in vacuo, and spiro(indene-1,4'-piperidine) (190 mg, 77.1%) was obtained as a white amorphous solid.

¹H-NMR (400 MHz, CDCl₃) δ; 1.35 (d, J= 13.0 Hz, 2H), 2.05-2.13 (m, 2H), 2.98-3.05 (m, 2H), 3.21-3.27 (m, 2H), 6.76 (d, J= 5.9 Hz, 1H), 6.89 (d, J= 5.9 Hz, 1H), 7.19-7.26 (m, 2H), 7.32 (d, J= 6.6 Hz, 1H), 7.39 (d, J= 6.6 Hz, 1H).

### Process 3: Preparation of 1-[Spiro(indene-1,4'-piperidine)-1'-yl]-2-[2-(trifluorometh yl)phenoxy]ethanone

1-[Spiro(indene-1,4'-piperidine)-1'-yl]-2-[2(trifluo romethyl)phenoxy]ethanone was produced by the following method.

A solution of spiro(indene-1,4'-piperidine)(190 mg, 1.03 mmol) and 2-trifluoromethylphenoxy acetate (220 mg, 1.00 mmol) in dichloromethane (3 mL) was added with triethylamine (203 mg, 2.01 mmol) and PyBOP (520 mg, 1.00 mmol) at room temperature. The resultant was stirred at the same temperature for 4 hours. The reaction solution was added with water, and extracted with chloroform. The organic layer was dried over anhydrous sodium sulfate, concentrated in vacuo, and the resultant residue was purified by silica-gel chromatography (hexane : ethyl acetate = 2 : 1), and 1-[spiro(indene-1,4'-piperidine)-1'-yl]-2-[2-(trifluorometh yl)phenoxy]ethanone(251 mg, 64.8%) was obtained as a colorless oil.

¹H-NMR (400 MHz, CDCl₃) δ; 1.36-1.41 (m, 2H), 1.92-2.00 (m, 2H), 3.06 (m, 1H), 3.45 (m, 1H), 4.25 (d, J= 13.9 Hz, 1H), 4.62 (d, J= 13.9 Hz, 1H), 4.85 (d, J= 13.4 Hz, 1H), 4.90 (d, J= 13.4 Hz, 1H), 6.80 (d, J= 5.6 Hz, 1H), 6.84 (d, J= 5.6 Hz, 1H), 7.10 (t, J= 7.3 Hz, 1H), 7.15-7.26 (m, 4H), 7.32 (d, J= 7.3 Hz, 1H), 7.54 (t, J= 7.3 Hz, 1H), 7.61 (d, J= 7.3 Hz, 1H).

IR (ATR); 1648, 1608, 1497, 1461, 1322, 1130, 1118, 1038, 753 cm⁻¹.

EI-MS m/z; 387 (M⁺).

### Example 2

### Preparation of 1-[spiro(indene-1,4'-piperidine)-1'-yl]-2-(o-tolyoxy)ethano ne:

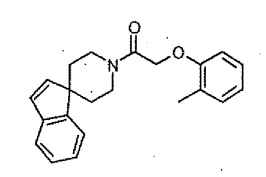

### Process 1: Preparation of 2-bramo-1-[spiro(indene-1,4'-piperidine)-1'-yl]ethanone

2-Bromo-1-[spiro(indene-1,4'-piperidine)-1'-yl]ethano ne was produced by the following method.

Under an argon atmosphere, a solution of spiro(indene-1,4'-piperidine)(296 mg, 1.60 mmol) in dichloromethane (15 mL) was added with sodium hydrogen carbonate (268 mg, 3.20 mmol) and bromoacetylbromide (323 mg, 1.6 mmol) at room temperature, and the resultant was stirred at the same temperature for 1 hour. The reaction solution was added with water, and extracted with chloroform. The organic layer was dried over anhydrous sodium sulfate, concentrated in vacuo, and the resultant residue was purified by silica-gel chromatography (ethyl acetate). 2-Bromo-1-[spiro(indene-1,4'-piperidine)-1'-yl]ethanone (429 mg, 87.5%) was obtained as a yellow amorphous solid.

¹H-NMR (400 MHz, CDCl₃) δ; 1.40-1.48 (m, 2H), 2.05 (m, 1H), 2.15 (m, 1H), 3.09 (m, 1H), 3.49 (m, 1H), 3.92-4.01 (m, 3H), 4.64 (m, 1H), 6.83 (d, J= 5.9 Hz, 1H), 6.85 (d, J= 5.9 Hz, 1H), 7.21-7.36 (m, 4H).

### Process 2: Preparation of 1-[spiro(indene-1,4'-piperidine)-1'-yl]-2-(o-tolyloxy)ethan one

1-[Spiro(indene-1,4'-piperidine)-1'-yl]-2-(o-tolyloxy )ethanone was produced by the following method.

A solution of 2-bromo-1-[spiro(indene-1,4'-piperidine)-1'-yl]ethanone (40.0 mg, 0.131 mmol) in N,N-dimethylformamide (2 mL) was added with potassium carbonate (54.3 mg, 0.393 mmol) and o-cresol (14.2 mg, 0.131 mmol) at room temperature, and the resultant was stirred at the same temperature for 40 hours. The reaction solution was added with water, and extracted with diethylether. The organic layer was dried over anhydrous sodium sulfate, concentrated in vacuo, and the resultant residue was purified by silica-gel chromatography (hexane : ethylacetate = 2 : 1). 1-[Spiro(indene-1,4'-piperidine)-1'-yl]-2-(o-tolyloxy)ethan one (36.1 mg, 82.6%)was obtained as a colorless oil.

¹H-NMR (400 MHz, CDCl₃) δ; 1.38 (d, J= 13.7 Hz, 2H), 1.94-2.03 (m. 2H), 2.27 (s, 3H), 3.05 (m, 1H), 3.43 (m, 1H), 4.21 (d, J= 13.9 Hz, 1H), 4.66 (d, J= 13.9 Hz, 1H), 4.76 (d, J= 13.0 Hz, 1H), 4.81 (d, J= 13.0 Hz, 1H), 6.80 (d, J= 5.8 Hz, 1H), 6.85 (d, J= 5.8 Hz, 1H), 6.91-6.95 (m, 2H), 7.14-7.27 (m, 5H), 7.33 (m, 1H).

IR (ATR); 2921, 1652, 1602, 1492, 1464, 1365, 1312, 1242, 1125, 1067, 1015, 781 cm⁻¹.

EI-MS m/z; 333 (M⁺).

### Example 3

### Preparation of 2-(2-ethylphenoxy)-1-[spiro(indene-1,4'-piperidine)-1'-yl]e thanone

The reaction and treatment were conducted in a similar manner to Process 2 of Example 2 using 2-ethylphenol in place of o-cresol, and 2-(2-ethylphenoxy)-1-[spiro(indene-1,4'-piperidine)-1'-yl]e thanone was obtained as a colorless oil.

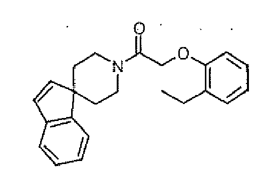

¹H-NMR (400 MHz, CDCl₃) δ; 1.21 (t, J= 7.6 Hz, 3H), 1.38 (d, J= 13.7 Hz, 2H), 1.95-2.05 (m, 2H), 2.69 (q, J= 7.6 Hz, 2H), 3.06 (m, 1H), 3.44 (m, 1H), 4.21 (d, J= 13.7 Hz, 1H), 4.67 (d, J= 13.7 Hz, 1H), 4.76 (d, J= 13.2 Hz, 1H), 4.81 (d, J= 13.2 Hz, 1H), 6.81 (d, J= 5.8 Hz, 1H), 6.85 (d, J= 5.8 Hz, 1H), 6.92-6.99 (m, 2H), 7.18-7.26 (m, 5H), 7.33 (d, J= 7.1 Hz, 1H).

IR (ATR); 2936, 1655, 1599, 1491, 1458, 1366. 1211, 1178, 1126, 1072, 1019, 753 cm⁻¹.

EI-MS m/z; 347 (M⁺).

### Example 4

### Preparation of 2-(2-iodophenoxy)-1-[spiro(indene-1,4'-piperidine)-1'-yl]et hanone

The reaction and treatment were conducted in a similar manner to Process 2 of Example 2 using 2-iodophenol in place of o-cresol, and 2-(2-iodophenoxy)-1-[spiro(indene-1,4'-piperidine)-1'-yl]et hanone was obtained as a colorless oil.

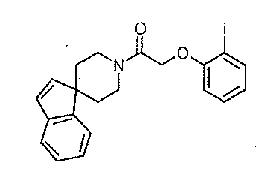

¹H-NMR (400 MHz, CDCl₃) δ; 1.34-1.44 (m, 2H), 1.91-2.08 (m, 2H), 3.04 (m, 1H), 3.46 (m, 1H), 4.27 (d, J= 13.7 Hz, 1H), 4.63 (d, J= 13.7 Hz, 1H), 4.81 (d, J= 13.2 Hz, 1H), 4.87 (d, J= 13.2 Hz, 1H), 6.76-6.81 (m, 2H), 6.84 (d, J= 5.9 Hz, 1H), 6.98 (m, 1H), 7.13-7.26 (m, 3H), 7.32-7.36 (m, 2H), 7.80 (dd, J= 1.7, 7.8 Hz, 1H).

IR (ATR); 2918, 1644, 1581, 1470, 1439, 1367, 1272, 1213, 1123, 1016, 749 cm⁻¹.

EI-MS m/z; 445 (M⁺).

### Example 5

### Preparation of ethyl 2-[2-oxo-2-[spiro(indene-1,4'-piperidine)-1'-yl]ethoxy]benz oate

The reaction and treatment were conducted in a similar manner to Process 2 of Example 2 using ethyl salicylate in place of o-cresol, and ethyl 2-[2-oxo-2-[spiro(indene-1,4'piperidine)-1'-yl]ethoxy]benz oate was obtained as a colorless oil.

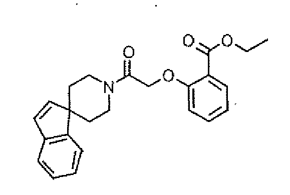

¹H-NMR (400 MHz, CDCl₃) δ; 1.32-1.39 (m, 5H), 1.90-1.99 (m, 2H), 3.03 (m, 1H), 3.45 (m, 1H), 4.29-4.37 (m, 3H), 4.62 (d, J= 13.7 Hz, 1H), 4.86 (d, J= 13.5 Hz, 1H), 4.91 (d, J= 13.5 Hz, 1H), 6.80 (d, J= 5.8 Hz, 1H), 6.84 (d, J= 5.8 Hz, 1H), 7.07 (m, 1H). 7.13-7.26 (m, 4H), 7.33 (m, 1H), 7.50 (m, 1H), 7. 83 (dd, J=2.0, 7.2 Hz, 1H).

IR (ATR) ; 2939, 1722, 1646, 1490, 1453, 1302, 1250, 1083, 1012, 752 cm⁻¹.

EI-MS m/z; 391 (M⁺).

### Example 6

### Preparation of 1-[spiro(indene-1,4'-piperidine)-1'-yl]-2-[3-trifluoromethy 1]phenoxy]ethanone

The reaction and treatment were conducted in a similar manner to Process 2 of Example 2 using 3-(trifluoromethyl)phenol in place of o-cresol, and 1-[spiro(indene-1,4'-piperidine)-1'-yl]-2-[3-trifluoromethy 1]phenoxy]ethanone was obtained as a colorless oil.

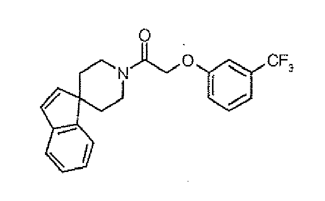

¹H-NMR (400 MHz, CDCl₃) δ; 1.38-1.45 (m, 2H), 1.94-2.08 (m, 2H), 3.08 (m, 1H), 3.46 (m, 1H), 4.08 (d, J= 13.7 Hz, 1H), 4.65 (d, J= 13.7 Hz, 1H), 4.81 (d, J= 13.2 Hz, 1H), 4.84 (d, J= 13.2 Hz, 1H), 6.82 (d, J= 5. 7 Hz, 1H), 6.85 (d, J= 5.7 Hz, 1H), 7.19-7.29 (m, 6H), 7.34 (m, 1H), 7.44 (m, 1H).

IR (ATR); 2936, 1654, 1455, 1327, 1167, 1124, 1066, 753 cm⁻¹.

EI-MS m/z; 387 (M⁺).

### Example 7

### Preparation of 2-(4-methoxyphenoxy)-1-[spiro(indene-1,4'-piperidine)-1'-yl ]ethanone

The reaction and treatment were conducted in a similar manner to Process 2 of Example 2 using 4-methoxyphenol in place of o-cresol, and 2-(4-methoxyphenoxy)-1-[spiro(indene-1,4'-piperidine)-1'-yl ]ethanone was obtained as a colorless oil.

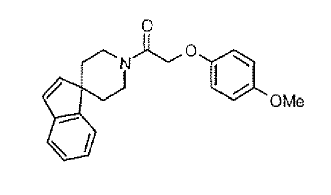

¹H-NMR (400 MHz, CDCl₃) δ; 1.37-1.40 (m, 2H), 1.94-2.07 (m, 2H), 3.05 (m, 1H), 3.44 (m, 1H), 3.79 (s, 3H), 4.12 (m, 1H), 4.64 (d, J= 13.7 Hz, 1H), 4.71 (d, J= 13.2 Hz, 1H), 4.74 (d, J= 13.2 Hz, 1H), 6.81 (d, J= 5.8 Hz, 1H), 6.84-6.88 (m, 3H), 6.93 (m, 1H), 6.95 (m, 1H), 7.20 (m, 2H), 7.26 (m, 1H), 7.33 (m, 1H).

IR (ATR); 2937, 1734, 1646, 1507, 1463, 1226, 1208, 1032, 1012, 827, 753 cm⁻¹.

EI-MS m/z; 349 (M⁺).

### Example 8

### Preparation of 2-(biphenyl-4-yloxy)-1-[spiro(indene-1,4'-piperidine)-1'-yl ]ethanone

The reaction and treatment were conducted in a similar manner to Process 2 of Example 2 using 4-phenylphenol in place of o-cresol, and 2-(biphenyl-4-yloxy)-1-[spiro(indene-1,4'-piperidine)-1'-yl ]ethanone was obtained as a colorless oil.

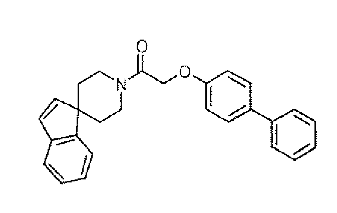

¹H-NMR (400 MHz, CDCl₃) δ; 1.36-1.41 (m, 2H), 1.93-2.05 (m, 2H), 3.05 (m, 1H), 3.44 (m, 1H), 4.16 (d, J= 13.9 Hz, 1H), 4.66 (d, J= 13.9 Hz, 1H), 4.80 (d, J= 13.2 Hz, 1H), 4.84 (d, J= 13.2 Hz, 1H), 6.80 (d, J= 5.6 Hz, 1H), 6.84 (d, J= 5.6 Hz, 1H), 7.05-7.09 (m, 2H), 7.15-7.27 (m, 3H), 7.31-7.34 (m, 2H), 7.40-7.44 (m, 2H), 7.54-7.57 (m, 4H).

IR (ATR); 2934, 1665, 1647, 1517, 1485, 1271, 1230, 1212, 1181, 1075, 837, 753 cm⁻¹.

EI-MS m/z; 395 (M⁺).

### Example 9

### Preparation of 2-(2,3-dichlorophenoxy)-1-[spiro(indene-1,4'-piperidine)-1' -yl]ethanone

The reaction and treatment were conducted in a similar manner to Process 2 of Example 2 using 2,3-dichlorophenol in place of o-cresol, and 2-(2,3-dichlorophenoxy)-1-[spiro(indene-1,4'-piperidine)-1' -yl]ethanone was obtained as a colorless oil.

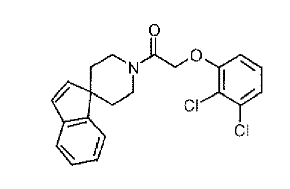

¹H-NMR (400 MHz, CDCl₃) δ; 1.36-1.45 (m, 2H), 1.92-2.07 (m, 2H), 3.05 (m, 1H), 3. 46 (m, 1H), 4.20 (d, J= 13.7 Hz, 1H), 4.62 (d, J= 13.7 Hz, 1H), 4.84 (d, J= 13.4 Hz, 1H), 4.89 (d, J= 13.4 Hz, 1H), 6.81 (d, J= 5. 8 Hz, 1H), 6.84 (d, J= 5.8 Hz, 1H), 7.00 (dd, J= 2.0, 7.8 Hz, 1H), 7.14-7.28 (m, 5H), 7.33 (m, 1H).

IR (ATR); 2930, 1647, 1583, 1478, 1454, 1430, 1260, 1242, 1081, 1013, 772, 751 cm⁻¹

EI-MS m/z; 388 (M⁺).

### Example 10

### Preparation of 1-[2,3-dihydrospiro(indene-1,4'-piperidine)-1'-yl]-2-[2-(tr ifluoromethyl)phenoxy]ethanone

1-[2,3-Dihydrospiro(indene-1,4'-piperidine)-1'-yl]-2-[2-(trifluoromethyl)phenoxy]ethanone was produced by the following method.

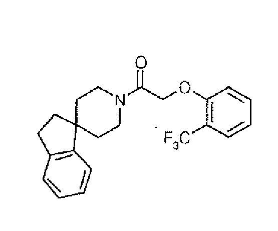

A solution of 1-[spiro(indene-1,4'-piperidine)-1'-yl]-2-[2-(trifluorometh 1)phenoxy]ethanone (20.9 mg, 0.054 mmol) in ethanol (2 mL) was added with 10% palladium carbon (catalyst amount), and the resultant was stirred at room temperature for 17 hours under hydrogen atmosphere. The reaction solution was filtered using celite, concentrated in vacuo, and 1-[2,3-dihydrospiro(indene-1,4'-piperidine)-1'-yl]-2-[2-tri fluoromethyl]phenoxy]ethanone (18.5 mg, 88.0%) was obtained as a colorless oil.

¹H-NMR (400 MHz, CDCl₃) δ; 1.56-1.59 (m, 2H), 1.70-1.78 (m, 2H), 2.06-2.10 (m, 2H), 2.83-2.95 (m, 3H), 3.29 (m, 1H), 4.12 (d, J= 13.7 Hz, 1H), 4.55 (d, J= 13.7 Hz, 1H), 4.82 (d, J= 13.4 Hz, 1H), 4.87 (d, J= 13.4 Hz, 1H), 6.97 (m, 1H), 7.08 (t, J= 7.6 Hz, 1H), 7.15-7.21 (m, 4H), 7.52 (t, J= 7.6 Hz, 1H), 7.60 (d, J= 7.6 Hz, 1H).

IR (ATR); 1648, 1608, 1496, 1322, 1140, 1118, 1038, 755 cm⁻¹.

EI-MS m/z; 389 (M⁺).

### Example 11

### Preparation of 1-[2-hydroxy-2,3-dihydrospiro(indene-1,4'-piperidine)-1'-yl ]-2-[2-(trifluoromethyl)phenoxy]ethanone

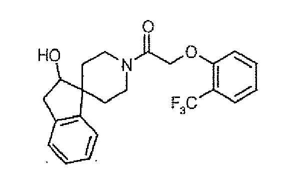

### Process 1: Preparation of 1-[2,3-oxa-2,3-dihydrospiro(indene-1,4'-piperidine)-1'-yl]-2-[2-(trifluoromethyl)phenoxy]ethanone

1-[2,3-Oxa-2,3-dihydrospiro(indene-1,4'-piperidine)-1 '-yl]-2-[2-(trifluoromethyl)phenoxy]ethanone was produced by the following method.

A solution of 1-[spiro(indene-1,4'-piperidine)-1'-yl]-2-[2-(trifluorometh yl)phenoxy]ethanone (200 mg, 0.54 mmol) in dichloromethane (4 mL) was added with pyridine (10.3 mg, 0.13 mmol), methyltrioxorhenium (2.6 mg, 0.01 mmol) and 30% hydrogen peroxide solution (1 mL) at room temperature, and the resultant was stirred at the same temperature for 15 hours. The reaction solution was added with water, and extracted with ethyl acetate. The organic layer was dried over anhydrous sodium sulfate, concentrated in vacuo, and the resultant residue was purified by silica-gel chromatography (hexane : ethyl acetate = 1 : 1). 1-[2,3-Oxa-2,3-dihydrospiro(indene-1,4'-piperidine)-1'-yl]-2-[2-(trifluoromethyl)phenoxy]ethanone (157 mg, 72.3%) was obtained as a colorless oil.

¹H-NMR (400 MHz, DMSO-*d*₆) δ; 1.47-1.98 (m, 4H), 3.05-3.50 (br, 2H), 3.80-4.30 (br, 2H), 4.31 (d, J= 3.0 Hz, 1H), 4.35 (d, J= 3.0 Hz, 1H), 5.01 (s, 2H), 7.08-7.30 (m, 5H), 7.50 (d, J= 6.5 Hz, 1H), 7.57-7.63 (m, 2H).

### Process 2: Preparation of 1-[2-hydroxy-2,3-dihydraspiro(indene-1,4'-piperidine)-1'-yl ]-2-[2-(trifluoromethyl)phenoxy]ethanone

1-[2-Hydroxy-2,3-dihydrospiro(indene-1,4'-piperidine) -1'-yl]-2-[2-(trifluoromethyl)phenoxy]ethanone was produced by the following method.

A solution of 1-[2,3-oxa-2,3-dihydrospiro(indene-1,4'-piperidine)-1'-yl]-2-[2-(trifluoromethyl)phenoxy]ethanone (88.6 mg, 0.22 mmol) in 1,4-dioxane (5 mL) was added with ammonium formate (20.0 mg, 0.31 mmol) and 10% palladium carbon (catalyst amount) at room temperature, and the resultant was stirred at 80°C for 2 hours. The reaction solution was filtered using celite, concentrated in vacuo, and the resultant residue was purified by silica-get chromatography (hexane : ethyl acetate = 1 : 3). 1-[2-Hydroxy-2,3-dihydrospiro(indene-1,4'-piperidine)-1'-yl ]-2-[2-(trifluoromethyl)phenoxy]ethanone (64.3 mg, 72.1%) was obtained as a colorless oil.

¹H-NMR (400 MHz, DMSO-*d*₆) δ; 1.55-1.78 (m, 3H), 2.03 (m, 1H), 2.75 (dd. J= 3.2, 16.5 Hz, 1H), 3.16 (dd, J= 5.7, 16.5 Hz, 1H), 3.21-3.45 (br, 2H), 3.80-4.05 (br, 1H), 4.36 (m, 1H), 4.74 (d, J= 5.1 Hz, 1H), 4.97 (s, 2H), 7.07-7.20 (m, 6H), 7.56-7.62 (m, 2H).

IR (ATR) ; 3410, 1640, 1608, 1496, 1460, 1322, 1117, 1038, 756 cm⁻¹.

EI-MS m/z; 405 (M⁺).

### Example 12

### Preparation of 1'-[2-[2-(trifluoromethyl)phenoxy]acetyl]spiro(indene-1,4'-piperidine)-2(3H)-one

1'-[2-[2-(Trifluoromethyl)phenoxy]acetyl]spiro(indene -1,4'-piperidine)-2(3H)-one was produced by the following method.

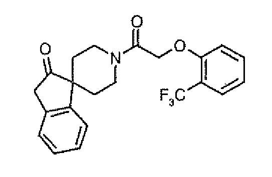

A solution of 1-[2-hydroxy-2,3-dihydrospiro(indene-1,4'-piperidine)-1'-yl ]-2-[2-(trifluoromethyl)phenoxy]ethanone (30.0 mg, 0.074 mmol) in dichloromethane (3 mL) was added with molecular sieves 4A (powder) (30.0 mg) and PCC (32.0 mg, 0.14 mmol) at 0°C, and the resultant was stirred at the same temperature four 1 hour. The reaction, solution was diluted with diethyl ether, purified by silica-gel chromatography (diethyl ether), and 1'-[2-[2-(trifluoromethyl)phenoxy]acetyl]spiro(indene-1,4'-piperidine)-2(3H)-one (23.5 mg, 78.7%) was obtained as a colorless oil.

¹H-NMR (400 MHz, CDCl₃) δ; 1.65-1.75 (m, 4H), 3.34 (m, 1H), 3.53 (s, 2H), 3.76 (m, 1H), 3.97 (d, J= 13.7 Hz, 1H), 4.34 (d, J= 13.7 Hz, 1H), 4.72 (d, J= 13.2 Hz, 1H), 4.83 (d, J= 13.2 Hz, 1H), 6.91 (m, 1H), 7.02 (t, J= 7.6 Hz, 1H), 7.10 (d, J= 7.6 Hz, 1H), 7.19-7.25 (m, 3H), 7.47 (t, J= 7.6 Hz, 1H), 7.53 (d, J= 7.6 Hz, 1H).

IR (ATR); 1739, 1647, 1497, 1460, 1322, 1260, 1037, 798 cm⁻¹.

EI-MS m/z; 403 (M⁺).

### Example 13

### Preparation of 1'-2-[2-(trifluoromethyl)phenoxy]acetyl]spiro(indene-1,4'-p iperidine)-3(2H)-one

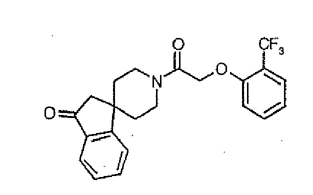

### Process 1: Preparation of t-butyl 3-oxo-2,3-dihydrospriro(indene-1,4'-piperidine)-1'-carboxyl ate t-Butyl

3-oxo-2,3-dihydrospriro(indene-1,4'-piperidine)-1'-carboxyl ate was produced by the following method.

Under an argon atmosphere, a solution of t-butyl spiro (indene-1,4'-piperidine)-1'-carboxylate (514 mg, 1.80 mmol) in tetrahydrofuran (5 mL) was added with a solution of 9-borabicyclo[3.3.1]nonane in tetrahydrofuran (0.5M, 10.5 mL)) at room temperature, and the resultant was stirred at the same temperature for 24 hours. The reaction solution was concentrated in vacuo, dissolved into dichloromethane (8 mL), and added with pyridinium chlorochromate (2.30 g, 10.7 mmol) at 0°C. The resultant was stirred for 2 hours by heating under reflux. The reaction solution was filtered using celite, and concentrated in vacuo. The resultant residue was purified by silica-gel chromatography (hexane : ethylacetate = 4 : 1), and t-butyl 3-oxo-2,3-dihydrospriro(indene-1,4'-piperidine)-1'-carboxyl ate (23.5 mg, 78.7%) was obtained as a colorless oil.

¹H-NMR (400 MHz, CDCl₃) δ; 1.46-1.57 (m, 2H), 1.50 (s, 9H), 1.96-2.01 (m, 2H), 2.64 (s, 2H), 2.80-2.89 (m, 2H), 4.20-4.26 (br, 2H), 7.42 (t, J= 7.3 Hz, 1H), 7.49 (d, J=7.3 Hz, 1H), 7.65 (t, J= 7.3 Hz, 1H), 7.74 (d, J= 7.3 Hz, 1H).

Process 2: The reaction and treatment were conducted in a similar manner to Processes 2 and 3 of Example 1 using t-butyl 3-oxo-2,3-dihydrospiro(indene-1,4'-piperidine)-1'-carboxyla te in place of t-butyl spiro(indene-1,4'-piperidine)-1'-carboxylate, and the title compound was obtained as a white amorphous solid.

¹H-NMR (400 MHz, CDCl₃) δ; 1.57 (d, J= 13.7 Hz, 2H), 1.89-1.97 (m, 2H), 2.66 (s, 2H), 2.77 (m, 1H), 3.23 (m, 1H), 4.27 (m, 1H), 4.70 (m, 1H), 4.82 (d, J= 13.2 Hz, 1H), 4.90 (d, J= 13.2 Hz, 1H), 7.10 (t, J= 7.6 Hz, 1H), 7.16 (d, J= 7.6 Hz, 1H), 7.30 (d, J= 7.6 Hz, 1H), 7.41 (t, J= 7.6 Hz, 1H), 7.55 (t, J= 7.6 Hz, 1H), 7.61-7.65 (m, 2H), 7.73 (d, J= 7.6 Hz, 1H).

IR (ATR); 1712, 1649, 1607, 1497, 1461, 1322, 1118, 1038, 758 cm⁻¹.

EI-MS m/z; 403 (M⁺).

### Example 14

### Preparation of 1-[spiro(cyclopenta[b]pyridine-7,4'-piperidine)-1'-yl]-2-[2 -(trifluoromethyl)phenoxy]ethanone

The reaction and treatment were conducted in a similar manner to Example 1 using 7H-cyclopenta[b]pyridine synthesized by the method described in WO2007/028638 in place of indene, and 1-[spiro(cyclopenta[b]pyridine-7,4'-piperidine)-1'-yl]-2-[2 -(trifluoromethyl)phenoxy]ethanone was obtained as a colorless oil.

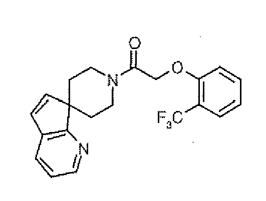

¹H-NMR (400 MHz, CDCl₃) δ; 1.51-1.60 (m, 2H), 1.93-2.02 (m, 2H), 3.51 (m, 1H), 3.65 (m, 1H), 4.33 (m, 1H), 4.45 (m, 1H). 4.82 (d, J= 13.7 Hz, 1H), 4.89 (d, J= 13.7 Hz, 1H), 6.71 (d, J= 6.1 Hz, 1H), 6.79 (d, J= 6.1 Hz, 1H), 7.07 (t, J= 7.6 Hz, 1H), 7.12-7.20 (m, 2H), 7.52 (t, J= 7.6 Hz, 1H), 7.56-7.60 (m, 2H), 8.32 (m, 1H).

IR (ATR); 1648, 1609, 1497, 1460, 1322, 1271, 1131, 1118, 1038, 754 cm⁻¹.

ET-MS m/z; 388 (M⁺).

### Example 15

### Preparation of 1-[5,6-dihydrospiro(cyclopenta[b]pyridine-7,4'-piperidine)-1'-yl]-2-[2-(trifluoromethyl)phenoxy]ethanone

The reaction and treatment were conducted in a similar manner to Example 10 using 1-[spiro(cyclopenta[b]pyridine-7,4'-piperidine)-1'-yl]-2-[2 -(trifluoromethyl)phenoxy]ethanone in place of 1-[spiro(indene-1,4'-piperidine)-1'-yl]-2-[2-(trifluorometh yl)phenoxy]ethanone, and the title compound was obtained as a colorless oil.

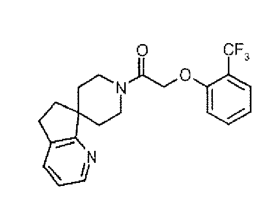

¹H-NMR (400 MHz, CDCl₃) δ; 1.52-1.62 (m, 2H), 2.04-2.26 (m, 4H), 2.97-3.07 (m, 3H), 3.37 (m, 1H), 4.14 (d, J= 13.4 Hz, 1H), 4.48 (d, J= 13.4 Hz, 1H), 4.80 (d, J= 13.7 Hz, 1H), 4.90 (d, J= 13.7 Hz, 1H), 7.04 (t, J= 7.8 Hz, 1H), 7.15 (d, J= 7.8 Hz, 1H), 7.21 (dd, J= 4.5, 7.8 Hz, 1H), 7.50 (t, J= 7.8 Hz, 1H), 7.56 (d, J= 7.8 Hz, 1H), 7.66 (d, J= 7.8 Hz, 1H), 8.41 (d, J= 4.6 Hz 1H).

IR (ATR); 1647, 1608, 1495, 1322, 1234, 1122, 1107, 1074, 1038, 762 cm⁻¹.

EI-MS m/z; 390 (M⁺).

### Example 16

### Preparation of 1-[2-methylspiro(indene-1,4'-piperidine)-1'-yl]-2-[2-(trifl uoromethyl)phenoxy]ethanone

The reaction and treatment were conducted in a similar manner to Example 1 using 2-methylindene in place of indene, and the title compound was obtained as a colorless oil.

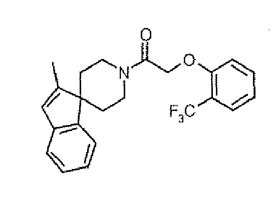

¹H-NMR (400 MHz, CDCl₃) δ; 1.27 (d, J= 13.4 Hz, 2H), 1.80 (s, 3H), 1.85-1.90 (m, 2H), 3.44 (t, J= 13.6 Hz, 1H), 3.83 (t, J= 13.6 Hz, 1H), 4.19 (d, J= 13.6 Hz, 1H), 4.58 (d, J= 13.6 Hz, 1H), 4.85 (d, J= 13.2 Hz, 1H), 4.92 (d, J= 13.2 Hz, 1H), 6.40 (s, 1H), 7.07-7.12 (m, 2H), 7.19 (d, J= 7.8 Hz, 1H), 7.25-7.27 (m, 2H), 7.54 (t, J= 7.8 Hz, 1H), 7.61-7.65 (m, 2H).

IR (ATR); 1647, 1608, 1497, 1460, 1321, 1118, 1038, 752 cm⁻¹.

EI-MS m/z; 401 (M⁺).

### Example 17

### Preparation of 1-[2-ethylspiro(indene-1,4'-piperidine)-1'-yl]-2-[2-(triflu oromethyl)phenoxy]ethanone

The reaction and treatment were conducted in a similar manner to Example 1 using 2-ethylindene was used in place of indene, and 1-[2-ethylspiro(indene-1,4'-piperidine)-1'-yl]-2-[2-(triflu oromethyl)phenoxy]ethanone was obtained as a colorless oil.

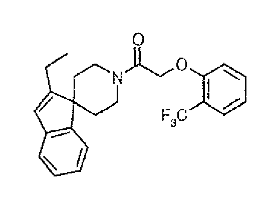

¹H-NMR (400 MHz, CDCl₃) δ; 1.18-1.28 (m, 5H), 1.85-2.12 (m, 4H), 3.41 (m, 1H), 3.81 (m, 1H), 4.18 (d, J= 13.9 Hz, 1H), 4.60 (d, J= 13.9 Hz, 1H), 4.85 (d, J= 13.4 Hz, 1H), 4.93 (d, J= 13.4 Hz, 1H), 6.43 (s, 1H), 7.07-7.12 (m, 2H), 7.19 (d, J= 7.8 Hz, 1H), 7.23-7.29 (m, 2H), 7.54 (t, J= 7.8 Hz, 1H), 7.59-7.65 (m, 2H).

IR (ATR); 1646, 1609, 1497, 1460, 1321, 1132, 1119, 1038, 751 cm⁻¹.

EI-MS m/z; 415 (M⁺).

### Example 18

### Preparation of 1-[2-propylspiro(indene-1,4'-piperidine)-1'-yl]-2-[2-(trifluoromethyl)phenoxy]ethanone

The reaction and treatment were conducted in a similar manner to Example 1 using 2-propylindene in place of indene, and the title compound was obtained as a light brown amorphous solid.

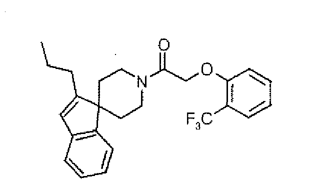

¹H-NMR (400 MHz, CDCl₃) δ; 0.97 (t, J= 7.3 Hz, 3H), 1.14-1.21 (m, 2H), 1.63-1.71 (m, 2H), 1.85-2.01 (m, 4H), 3.39 (m, 1H), 3.80 (m, 1H). 4.20 (m, 1H), 4.61 (m, 1H). 4.85 (d, J= 13.4 Hz, 1H), 4.93 (d, J= 13.4 Hz, 1H), 6.41 (s, 1H), 7.07-7.11 (m, 2H), 7.20 (d, J= 8.3 Hz, 1H), 7.23-7.28 (m, 2H), 7.54 (t, J= 8.3 Hz, 1H), 7.61-7.66 (m, 2H).

IR (ATR); 1647, 1608, 1497, 1459, 1321, 1130, 1118, 1038, 753 cm⁻¹.

EI-MS m/z; 429 (M⁺).

### Example 19

### Preparation of 1-[2-isopropylspiro(indene-1,4'-piperidine)-1'-yl]-2-[2-(tr ifluoromethyl)phenoxy]ethanone

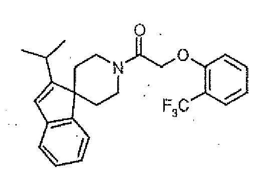

### Process 1: Preparation of 2-isopropylindene

2-Isopropylindene was produced by the following method.

Under an argon atmosphere, a solution of zinc chloride (3.80 g, 28.0 mmol) in tetrahydrofuran suspension (5 mL) was added with a solution of isopropyl magnesium bromide in tetrahydrofuran (0.78 M, 30.7 mL) at 0°C, and the resultant was stirred at the same temperature for 1 hour. Then a solution of tetrakistriphenyl phosphine palladium (800 mg, 0.70 mmol) and 2-bromoindene (3.87 g, 20.0 mmol) in tetrahydrofuran (10 mL) was added thereto, and the resultant was further stirred at the same temperature for 16 hours. The reaction solution was added with a saturated aqueous solution of ammonium chloride, and extracted with diethyl ether. The organic layer was dried over anhydrous sodium sulfate, concentrated in vacuo, and the resultant residue was purified by silica-gel chromatography (hexane). 2-Isopropylindene (2.25 g, 71.1%) was obtained as a colorless oil.

¹H-NMR (400 MHz, CDCl₃) δ; 1.22 (d, J= 6.6 Hz, 6H), 2.77 (septet, J= 6.6 Hz, 1H), 3.30 (s, 2H), 6.50 (s, 1H) 7.10-7.34 (m, 4H).

Process 2: The reaction and treatment were conducted in a similar manner to Example 1 using 2-isopropylindene in place of indene, and the title compound was obtained as a colorless oil.

¹H-NMR (400 MHz, CDCl₃) δ; 1.06 (d, J= 6.6 Hz, 3H), 1.09 (d, J=6.6Hz, 3H), 1.20-1.24 (m, 2H), 1.85-2.00 (m, 2H), 2.11 (septet, J= 6.6 Hz, 1H), 3.34 (m, 1H), 3.77 (m, 1H), 4.22 (m, 1H), 4.65 (m, 1H), 4.85 (d, J= 13.4 Hz, 1H), 4.94 (d, J= 13.4 Hz, 1H), 6.48 (s, 1H), 7.07-7.11 (m, 2H), 7.23-7.26 (m, 3H), 7.54 (t, J= 7.8 Hz, 1H), 7.61 (d, J= 7.8 Hz, 1H), 7.65 (d, J= 7.8 Hz, 1H).

IR (ATR); 1647, 1609, 1497, 1459, 1321, 1130, 1119, 1038, 752 cm⁻¹.

EI-MS m/z; 429 (M⁺).

### Example 20

### Preparation of 1-[2-cyclopropylspiro(indene-1,4'-piperidine)-1'-yl]-2-[2-( trifluoromethyl)phenoxy]ethanone

he reaction and treatment were conducted in a similar manner to Example 19 using cyclopropylmagnesium bromide in place of isopropylmagnesium bromide. 1-[2-Cyclopropylspiro(indene-1,4'-piperidine)-1'-yl]-2-[2-( trifluoromethyl)phenoxy]ethanone was obtained as a white amorphous solid.

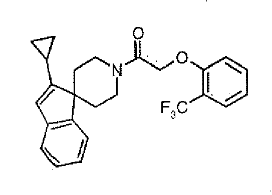

¹H-NMR (400 MHz, CDCl₃) δ; 0.51-0.59 (m, 2H), 0.77-0.84 (m, 2H), 1.14 (m, 1H), 1.26-1.32 (m, 2H), 2.00-2.11 (m, 2H), 3.47 (m, 1H), 3.82 (m, 1H), 4.22 (d, J= 13.9 Hz, 1H), 4.59 (d, J= 13.9 Hz, 1H), 4.86 (d, J= 13.7 Hz, 1H), 4.94 (d, J= 13.7 Hz, 1H), 6.13 (s, 1H), 7.05-7.11 (m, 2H), 7.20-7.29 (m, 3H), 7.53 (t, J= 7.9 Hz, 1H), 7.59-7.62 (m, 2H).

IR (ATR); 1646, 1609, 1497, 1460, 1321, 1130, 1118, 1038, 752 cm⁻¹.

EI-MS m/z; 427 (M⁺).

### Example 21

### Preparation of 1-[2-phenylspiro(indene-1,4'-piperidine)-1'-yl]-2-[2-(trifl uoromethyl)phenoxy]ethanone

The reaction and treatment were conducted in a similar manner to Example 1 using 2-phenylindene in place of indene, and 1-[2-phenylspiro(indene-1,4'-piperidine)-1'-yl]-2-[2--(trifl uoromethyl)phenoxy]ethanone was obtained as a white amorphous solid.

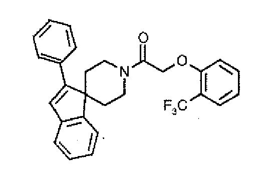

¹H-NMR (400 MHz, CDCl₃) δ; 1.44-1.52 (m, 2H), 2.09-2.22 (m, 2H), 3.49 (m, 1H), 3.85 (m, 1H), 4.11 (d, J= 13.6 Hz, 1H), 4.45 (d, J= 13.6 Hz, 1H), 4.79 (d, J= 13.4 Hz, 1H), 4.85 (d, J= 13.4 Hz, 1H), 6.76 (s, 1H), 7.06 (t, J=7.6Hz, 1H). 7.12 (d, J= 7.6 Hz, 1H), 7.17-7.33 (m, 7H), 7.38 (d, J= 7.6 Hz, 1H), 7.48 (t, J= 7.6 Hz, 1H), 7.57 (d, J= 7.6 Hz, 1H), 7.68 (d, J= 7.6 Hz, 1H).

IR (ATR); 1647, 1608, 1496, 1459, 1321, 1130, 1118, 1038, 754 cm⁻¹.

EI-MS m/z; 464 (M⁺).

### Example 22

### Preparation of 1-[2-chlorospiro(indene-1,4'-piperidine)-1'-yl]-2-[2-(trifl uoromethyl)phenoxy]ethanone

The reaction and treatment were conducted in a similar manner to Example 1 using 2-chloroindene in place of indene and 1-[2-chlorospiro(indene-1,4'-piperidine)-1'-yl]-2-[2-(trifluoromethyl)phenoxy]ethanone was obtained as a white amorphous solid.

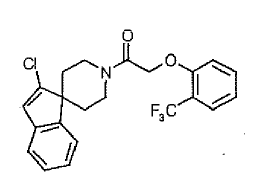

¹H-NMR (400 MHz, CDCl₃) δ; 1.42-1.50 (m, 2H), 1.97-2.05 (m, 2H), 3.55 (m, 1H), 3.83 (m, 1H), 4.23 (m, 1H), 4.50 (m, 1H), 4.90 (s, 2H), 6.66 (s, 1H), 7.09 (t, J= 7.6 Hz, 1H), 7.15-7.20 (m, 2H), 7.26-7.29 (m, 2H), 7.52-7.55 (m, 2H), 7.61 (d, J= 7.6 Hz, 1H).

IR (ATR); 1646, 1609, 1460, 1325. 1291, 1218, 1122, 1095, 1069, 1037, 758, 751 cm⁻¹.

EI-MS m/z; 421 (M⁺).

### Example 23

### Preparation of 1-[2-bromospiro(indene-1,4'-piperidi-ne)-1'-yl]-2-[2-(triflu oromethyl)phenoxy]ethanone

The reaction and treatment were conducted in a similar manner to Example 1 using 2-bromoindene in place of indene, and 1-[2-bromospiro(indene-1,4'-piperidine)-1'-yl]-2-[2-(triflu oromethyl)phenoxy]ethanone was obtained as a light brown oil.

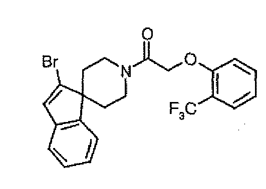

¹H-NMR (400 MHz, CDCl₃) δ; 1.34-1.42 (m, 2H), 1.95-2.03 (m, 2H), 3.48 (m, 1H), 3.82 (m, 1H), 4.27 (m, 1H), 4.58 (m, 1H), 4.90 (s, 2H), 6.84 (s, 1H), 7.08 (t, J= 7.6 Hz, 1H), 7.15-7.20 (m, 2H), 7.26-7.29 (m, 2H), 7.54 (t, J= 7.6 Hz, 1H), 7.60-7.62 (m, 2H).

IR (ATR); 1647, 1608, 1497, 1459, 1321, 1119, 1061, 1038, 751 cm⁻¹.

EI-MS m/z; 465 (M⁺).

### Example 24

### Preparation of 1-[3-methylspiro(indene-1,4'-piperidine)-1'-yl]-2-[2-(trifl uoromethyl)phenoxy]ethanone

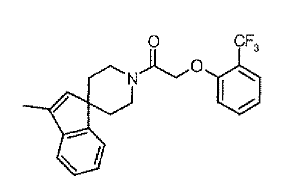

### Process 1: Preparation of t-butyl 3-hydroxy-3-methyl-2,3-dihydrospiro(indene-1,4'-(piperidine )-1'-carboxylate

t-Butyl 3-hydroxy-3-methyl-2,3-dihydrospiro(indene-1,4'-(piperidine )-1'-carboxylate was produced by the following method.

Under an argon atmosphere, a solution of to-butyl 3-oxo-2,3-dihydrospiro(indene-1,4'-plperidine)-1'-carboxyla te (30.0 mg, 0.10 mmol) in tetrahydrofuran (1 mL) was added with a solution of methyllithium in diethylether (1 M, 0.1 mL) at -78°C, and the resultant was stirred at the same temperature for 10 minutes. The reaction solution was added with saturated ammonium chloride aqueous solution, and extracted with diethyl ether. The organic layer was dried over anhydrous sodium sulfate, concentrated in vacuo, and the resultant residue was purified by silica-gel chromatography (hexane : ethyl acetate = 1 : 1). t-Butyl 3-hydroxy-3-methyl-2,3-dihydrospiro(indene-1,4'-(piperidine )-1'-carboxylate (16.2 mg, 51.0%) was obtained as a colorless oil.

¹H-NMR (400 MHz, CDCl₃) δ; 1.49 (s, 9H), 1.58 (s, 3H), 1.60-1.66 (m, 2H), 1.72-1.92 (m, 2H), 2.14 (d, J= 13.6 Hz, 1H), 2.34 (d, J= 13.6 Hz, 1H), 2.85-2.99 (br, 2H), 4.03-4.25 (br, 2H), 7.19 (d, J= 6.4 Hz, 1H), 7.26-7.34 (m, 3H).

### Process 2: Preparation of 3-methylspiro(indene-1,4'-piperidine)

3-Methylspiro(indene-1,4'-piperidine) was produced by the following method.

A solution of t-butyl 3-hydroxy-3-methyl-2,3-dihydrospiro(indene-1,4'-piperidine) -1'-carboxylate (16.2 mg, 0.051 mmol) in dichloromethane(1 mL) was added with trifluoroacetic acid (1 mL), and the resultant was stirred at room temperature for 15 hours. The reaction solution was concentrated in vacuo, and 3-methylspiro(indene-1,4'-piperidine) (7.4 mg, 72.8%) was obtained as a colorless oil.

¹H-NMR (400 MHz, CBCl₃) δ; 1.21-1.35 (m, 2H), 1.95-2.05 (m, 2H), 2.14 (s, 3H), 2.92-2.98 (m, 2H), 3.01-3.21 (m, 2H), 6.57 (s, 1H), 7.22-7.29 (m, 3H), 7.36 (d, J= 8.0 Hz, 1H).

Process 3: The reaction and treatment were conducted in a similar manner to Process 3 of Example 1 using 3-methylspiro(indene-1,4'-(piperidine) in place of spiro(indene-1,4'-piperidine), and 1-[3-methylspiro(indene-1,4'-piperidine)-1'-yl]-2-[2-(trifl uoromethyl)phenoxy]ethanone was obtained as a colorless oil.

¹H-NMR (400 MHz, CDCl₃) δ; 1.33-1.37 (m, 2H), 1.88-1.98 (m, 2H), 2.13 (s, 3H), 3.03 (m, 1H), 3.43 (m, 1H), 4.22 (m, 1H), 4.61 (m, 1H), 4.86 (d, J= 13.2 Hz, 1H), 4.90 (d, J= 13.2 Hz, 1H), 6.40 (s, 1H), 7.09 (t, J= 7.6 Hz, 1H), 7.14-7.31 (m, 5H), 7.54 (t, J= 7.6 Hz, 1H), 7.61 (d, J= 7.6 Hz, 1H).

IR (ATR); 1652, 1459, 1322, 1130, 1118, 1038, 755 cm⁻¹.

EI-MS m/z; 401 (M⁺).

### Example 25

### Preparation of 1-[2-methyl-2,3-dihydrospiro(indene-1,4'-piperidine)-1'-yl] -2-[2-(trifluoromethyl)phenoxy]ethanone

The reaction and treatment were conducted in a similar manner to Example 10 using 1-[2-methylspiro(indene-1,4'-piperidine)-1'-yl]-2-[2-(trifl uoromethyl)phenoxy]ethanone in place of 1-[spiro(indene-1,4'-piperidine)-1'-yl]-2-[2-(trifluorometh yl)phenoxy]ethanone, and 1-[2-methyl-2,3-dihydrospiro(indene-1,4'-piperidine)-1'-yl] -2-[2-(trifluoromethyl)phenoxy]ethanone was obtained as a white amorphous solid.

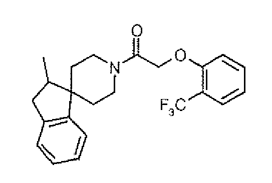

¹H-NMR (270 MHz, DMSO-d₆) δ; 0.91 (d, J= 6.5 Hz, 3H), 1.39-1.80 (m, 4H), 2.39-2.51 (m, 2H), 3.02-3.46 (m, 3H), 3.80-3.92 (br, 2H), 4.97 (s, 2H), 7.07-7.23 (m, 6H), 7.57-7.62 (m, 2H).

IR (ATR); 1651, 1608, 1460, 1321, 1118, 1038, 755 cm⁻¹.

EI-MS m/z; 403 (M⁺).

### Example 26

### Preparation of 1-[2-ethylspiro(indene-1,4'-piperidine)-1'-yl]-2-methyl-2-[ 2-(trifluoromethyl)phenoxy]propane-1-one

### Process 1: Preparation of 2-methyl-2-[2-(trifluoromethyl)phenoxy]propionic acid

2-Methyl-2-[2-(trifluoromethyl)phenoxy] propionic acid was produced by the following method.

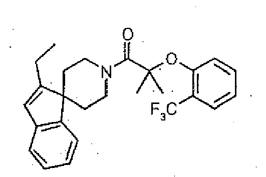

A solution of 2-(trifluoromethyl)phenol (3.20 g, 20.0 mmol) in acetone (20 mL) was added with 1,1,1-trichloro-2-methyl-2-propanol 0.5 hydrate (7.82 g, 40.0 mmol) and sodium hydroxide (3.20 g, 80.0 mmol) at 0°C, and the resultant was stirred at room temperature for 20 hours. The reaction solution was concentrated in vacuo, added with water, and washed with diethyl ether. The aqueous layer was neutralize with concentrated hydrochloric acid, and extracted with chloroform. The organic layer was dried over anhydrous sodium sulfate, concentrated in vacuo, and 2-methyl-2-[2-(trifluoromethyl)phenoxy] propionic acid (1.35 g, 27.2%) was obtained as a while amorphous solid.

¹H-NMR (400 MHz, CDCl₃) δ; 1.67 (s, 6H), 6.97 (d, J= 8.3 Hz, 1H), 7.10 (t, J= 8.3 Hz, 1H), 7.45 (t, J= 8.3 Hz, 1H), 7.60 (d, J= 8.3 Hz, 1H).

Process 2: The reaction and treatment were conducted in a similar manner to Example 1 using 2-ethylindene in place of indene, and 2-methyl-2-[trifluoromethylphenoxy]propionic acid in place of 2-trifluoromethyl phenoxy acetate, and 1-[2-ethylspiro(indene-1,4'-piperidine)-1'-yl]-2-methyl-2-[ 2-(trifluoromethyl)phenoxy]propane-1-one was obtained as a white amorphous solid.

¹H-NMR (400 MHz, CDCl₃) δ; 1.10 (t, J= 7.3 Hz, 3H), 1.19-1.32 (m, 2H), 1.54-1.85 (m, 4H), 1.77 (s, 6H), 3.38 (m, 1H), 3.69 (m, 1H), 4.66-4.72 (m, 2H), 6.34 (s, 1H), 7.00-7.07 (m, 3H), 7.19-7.24 (m, 2H), 7.48 (t, J= 7. 9 Hz, 1H), 7.57 (d, J= 7.9 Hz, 1H), 7.60 (d, J= 7.9 Hz, 1H).

IR (ATR); 1629, 1608, 1494, 1435, 1319, 1277, 1136, 1118, 1038, 750 cm⁻¹.

EI-MS m/z; 443 (M⁺).

### Test Example 1

### Human 11β-HSD1-, human 11β-HSD2- and mouse 11β-HSD1-inhibitory effect

### 1. Human 11β-HSD1-, human 11β-HSD2- and mouse 11β-HSD1-gene clonings and establishment of stably expressing cells

Human 11β-HSD-, human 11β-HSD2-, and mouse 11β-HSD1-gene clonings were conducted using as a template a reverse transcription product of human-liver RNA, human-kidney RNA (CELL APPLICATIONS), and mouse-kidney RNA respectively, by means of PCR cloning with reference to nucleotide sequences of Genbank Accession Nos. NM_005525, NM_000196, and NM 008288. The obtained PCR products of about 0.9 kbp, 1.2 kbp, and 0.9 kbp were subcloned into an expression vector pcDNA3.1+/Zeo (Invitrogen).

Human 11β-HSD1- and human 11β-HSD2-expressing vectors were transfected into human kidney-derived cell line, HEK293 cells, using a transfection reagent, jet PEI (Funakoshi). Mouse 1β-HSD1 was transfected to Chinese hamster ovary-derived cell line, CHO-K1 cells. Selection was conducted with 200-400 µg/mL of zeocine (Invitrogen) to provide stably expressing-cell clones. The stably expressing cells were suspended in buffer solution A (20 mmol/L Tris-HCl, pH 7.4, 250 mmol/L sucrose, 1 mmol/L EGTA, lmmol/L EDTA, mmol/L MgCl₂), sonicated, and then stored at -80° C.

### 2. Assay of enzyme inhibitory activity

An enzymatic reaction was conducted using a polystyrene 96-well plate. Each well was added with 1 µL of a test agent dissolved in DMSO and then diluted (0.003 to 3 mmol/L), and further added with 10 µL of cell lysate diluted to a concentration of 0.1 mg/mL to 0.4 mg/mL. Next, 90 µL of buffer solution A containing substrate (100 nmol/L cortisone or cortisol) and coenzyme (400 µmol/L NADPH or NAD+) was added and the mixture was incubated at 37°C for 1 hour. The enzymatic reaction was stopped by treating at 95°C for 3 minutes. Cortisol that was present in the reaction solution was determined by a competitive ELISA shown below.
Anti-rabbit IgG antibody (Chemi-con) diluted to 2 µg/mL with carbonate buffer solution (pH 9.6) was added in 100 µL each to a 96-well immune plate (Nunc) and immobilized by an incubation at 4°C overnight. 50 µL of enzymatic reaction solution was put onto the plates, and further, anti-cortisol antibody (Cosmo Bio) and HRP-labeled cortisol (Cosmo Bio), diluted with buffer solution B (25 mmol/L Tris-HCl pH 7.4, 137 mmol/L NaCl, 2.68 mmol/L KCl), were added in 50 µL respectively and incubated at 4°C overnight. After washed three times with buffer solution B containing 0.05% Tween 20, the plates were allowed to develop color by adding 100 µL of color reagent, TMB (Moss). The color reaction was stopped by 25 µL of 1 mol/L sulfuric acid and the absorbance was determined at 450 nm with a microplate reader (Molecular Device, VersaMax).

The values of human 11β-HSD1, human 11β-HSD2, and mouse 11β-HSD1 activities were subtracted from 100, and the resultant values were regarded as the respective 11β-HSD inhibition rates of example compounds. For each example compound, the value of 50% inhibitory concentration (IC₅₀) was calculated from 11β-HSD inhibition rates at plural concentrations, for 11β-HSD1 and 11β-HSD2 activities. The results are shown in tables 1 and 2.

**[Table 1] Enzyme inhibitory selectivity of human 11β-HSD1 and human 11β-HSD2**

| Example No. | IC₅₀(µM) | |
|---|---|---|
| | HSD1 | HSD2 |
| 1 | 0.029 | >30 |
| 3 | 0.72 | >30 |
| 4 | 0.11 | >30 |
| 10 | 0.025 | NA |
| 11 | 0.23 | NA |
| 12 | 0.077 | >30 |
| 13 | 1 | NA |
| 16 | 0.043 | NA |
| 17 | 0.048 | NA |
| 18 | 0.24 | NA |
| 19 | 0.34 | NA |
| 20 | 0.088 | NA |
| 22 | 0.030 | NA |
| 23 | 0.049 | NA |
| 24 | 0.066 | NA |
| 25 | 0.12 | NA |

As it is shown in Table 1, it has been confirmed that the compound of the present invention has an activity to strongly and selectively inhibit human 11β-HSD1.

### Test Example 2

### Enzyme inhibitory effect of human HSD1 and mouse HSD1

**[Table 2]**

| Example No. | IC₅₀(µM) | |
|---|---|---|
| | Human HSD1 | Mouse HSD1 |
| 1 | 0.029 | 0.72 |
| 10 | 0.025 | 1.6 |
| 16 | 0.043 | 0.63 |
| 17 | 0.048 | 0.56 |
| 22 | 0.030 | 2.0 |
| 23 | 0.049 | 1.4 |

For medicine development, it is required to make a dosage selection for clinical experiments by extrapolating data which have been accumulated with animal models to human. Sometimes, the difference of enzyme species becomes an issue for evaluating an inhibitor targeting a certain enzyme, such as the compound of the present invention. Specifically, as rodents such as mouse are generally used as an animal model, a compound having an inhibitory activity to mouse type enzyme as well as human type enzyme, has an advantageous property for evaluating usefulness as medicine. As it is shown in Table 2, the compound of the present invention has been confirmed to also have an inhibitory effect to mouse 11β-HSD1.

## Claims

1. A spiro compound represented by the following general formula (1) or salt thereof, or their solvate: 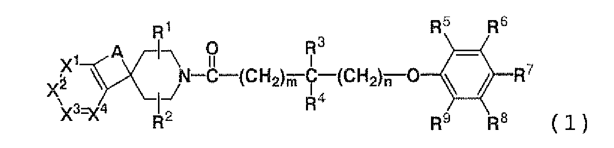 (wherein all of X¹, X², X³ and X⁴ are C-R¹⁰, or one of X¹, X², X³ and X⁴ is N, and the rest is C-R¹⁰, A represents the following formula (2) or (3): 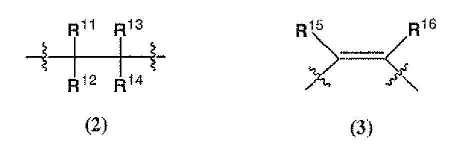 R₁, R₂, R₃, and R₄ are same or different and are a hydrogen atom, C₁₋₆ alkyl group, halo C₁₋₆ alkyl group, C₂₋₆ alkenyl group, C₂₋₆ alkynyl group, C₃₋₈ cycloalkyl group, C₁₋₆ alkoxy group, C₁₋₆ alkoxy-C₁₋₆ alkyl group, C₆₋₁₀ aryl group, C₆₋₁₀ aryloxy group, or 5- to 10-membered heteroaryl group; R⁵, R⁶,R⁷,R⁸,R⁹ and R¹⁰ are same or different, and are a hydrogen atom, halogen atom, C₁₋₆ alkyl group, halo C₁₋₆ alkyl group, C₂₋₆ alkenyl group, C₂₋₆ alkynyl group, C₃₋₈ cycloalkyl group, C₁₋₆ alkoxy group, halo C₁₋₆ alkoxy group, C₁₋₆ alkanoyl group, formyl group, nitro group, amino group, mono C₁₋₆ alkylamino group, diC₁₋₆ alkylamino group, cyano group, hydroxy group, carboxyl group, C₁₋₆ alkoxycarbonyl group, carbamoyl group, C₆₋₁₀ aryl group, 5- to 10-membered heteroaryl group, C₁₋₆ alkylthio group, C₁₋₆ alkylsulphonyl group, C₆₋₁₀ arylthio group, or C₆₋₁₀ arylsulphonyl group; or two adjacent R⁵∼R⁹ may bond to form C₁₋₆ alkylenedioxy group; R¹¹, R¹², R¹³, R¹⁴, R¹⁵ and R¹⁶ are same or different, and are a hydrogen atom, halogen atom, C₁₋₆ alkyl group, halo C₁₋₆ alkyl group, C₂₋₆ alkenyl group, C₂₋₆ alkynyl group, C₃₋₈ cycloalkyl group, C₆₋₁₀ aryl group, hydroxy group, or C₁₋₆ alkoxy group, or R¹¹, and R¹² or R¹³ and R¹⁴ may together bond with a same oxygen atom; m and n represent an integer of 0 to 6, and the sum of m and n represents an integer of 0 to 6).

2. The spiro compound or salt thereof, or their solvate according to claim 1, wherein at least one of R⁵, R⁶,R⁷,R⁸ and R⁹ is a halo C₁₋₆ alkyl group or C₆₋₁₀ aryl group.

3. The spiro compound or salt thereof, or their solvate according to claim 1, wherein the compound represented by the general formula (1) is a compound selected from the group consisting of:
1-[spiro(indene-1,4'-piperidine)-1'-yl]-2-[2-(trifluorometh yl)phenoxy]ethanone,
1-[spiro(indene-1,4'-plperidine)-1'-yl]-2-(o-tolyloxy)ethan one,
2-(2-ethylphenoxy)-1-[spiro(indene-1,4'-piperidine)-1'-yl]e thanone,
2-(2-iodophenoxy)-1-[spiro(indene-1,4'-piperidine)-1'-yl]et hanone,
ethyl
2-[2-oxo-2-[spiro(indene-1,4'-piperidine)-1'-yl]ethoxy]benz oate,
1-[spiro(indene-1,4'-piperidine)-1'-yl]-2-[3-(trifluorometh yl)phenoxy]ethanone,
2-(4-methoxyphenoxy)-1-[spiro(indene-1,4'-piperidine)-1'-yl ]ethanone,
2-(biphenyl-4-yloxy)-1-[spiro(indene-1,4'-piperidine)-1'-yl ]ethanone,
2-(2,3-dichlorophenoxy)-1-[spiro(indene-1,4'-piperidine)-1' -yl]ethanone,
1-[2,3-dihydrospiro(indene-1,4'-piperidine)-1'-yl]-2-[2-(tr ifluoromethyl)phenoxy]ethanone,
1-[2-hydroxy-2,3-dihydrospiro(indene-1,4'-piperidine)-1'-yl ]-2-[2-(trifluoromethyl)phenoxy]ethanone,
1'-[2-[2-(trifluoromethyl)phenoxy]acetyl]spiro(indene-1,4'-piperidine)-2(3H)-one,
1'-[2-[2-(trifluoromethyl)phenoxy]acetyl]spiro(indene-1,4'-piperidine)-3(2H)-one,
1-[spiro(cyclopenta[b]pyridine-7,4'-piperidine)-1'-yl]-2-[2 -(trifluoromethyl)phenoxy]ethanone,
1-[5,6-dihydrospiro(cyclopenta[b]pyridine-7,4'-piperidine)-1'-yl]-2-[2-(trifluoromethyl)phenoxy]ethanone,
1-[2-methylspiro(indene-1,4'-piperidine)-1'-yl]-2-[2-(trifl uoromethyl)phenoxy]ethanone,
1-[2-ethylspiro(indene-1,4'-piperidine)-1'-yl]-2-[2-(triflu oromethyl)phenoxy]ethanone,
1-[2-propylspiro(indene-1,4'-piperidine)-1'-yl]-2-[2-(trifl uoromethyl)phenoxy]ethanone,
1-[2-isopropylspiro(indene-1,4'-piperidine)-1'-yl]-2-[2-(tr ifluoromethyl)phenoxy]ethanone,
1-[2-cyclopropylspiro(indene-1,4'-piperidine)-1'-yl]-2-[2-( trifluoromethyl)phenoxy]ethanone,
1-[2-phenylspiro(indene-1,4'-piperidine)-1'-yl]-2-[2-(trifl uoromethyl)phenoxy]ethanone,
1-[2-chlorospiro(indene-1,4'-piperidine)-1'-yl]-2-[2-(trifl uoromethyl)phenoxy]ethanone,
1-[2-bromospiro(indene-1,4'-piperidine)-1'-yl]-2-[2-(triflu oromethyl)phenoxy]ethanone,
1-[3-methylspiro(indene-1,4'-piperidine)-1'-yl]-2-[2-(trifl uoromethyl)phenoxy]ethanone,
1-[2-methyl-2,3-dihydrospiro(indene-1,4'-piperidine)-1'-yl] -2-[2-(trifluoromethyl)phenoxy]ethanone, and
1-[2-ethylspiro(indene-1,4'-piperidine)-1'-yl]-2-methyl-2-[ 2-(trifluoromethyl)phenoxy]propan-1-one.

4. A pharmaceutical composition consisting of a spiro compound or salt thereof, or their solvate according to any one of claims 1 to 3, and a pharmaceutically acceptable carrier.

5. An inhibitor of 11β-hydroxysteroid dehydrogenase 1, comprising the spiro compound or salt thereof, or their solvate according to any one of claims 1 to 3 as an active ingredient.

6. An agent for preventing and/or treating diabetes, insulin resistance, diabetes complication, obesity, dyslipidemia, hypertension, fatty liver, or metabolic syndrome, which agent comprises the spiro compound or salt thereof, or their solvate according to any one of claims 1 to 3 as an active ingredient.

7. Use of the spiro compound or salt thereof, or their solvate according to any one of claims 1 to 3 for producing a formulation for inhibiting 11β-hydroxysteroid dehydrogenase 1.

8. Use of the spiro compound or salt thereof, or their solvate according to any one of claims 1 to 3 for producing a formulation for an agent for preventing and/or treating diabetes, insulin resistance, diabetes complication, obesity, dyslipidemia, hypertension, fatty liver, or metabolic syndrome.

9. A method for inhibiting 11β-hydroxysteroid dehydrogenase 1, which method comprises administering an effective amount of the spiro compound or salt thereof, or their solvate according to any one of claims 1 to 3.

10. A method for preventing and/or treating diabetes, insulin resistance, diabetes complication, obesity, dyslipidemia, hypertension, fatty liver, or metabolic syndrome, which method comprises administering an effective amount of the spiro compound or salt thereof, or their solvate according to any one of claims 1 to 3.
